(19)

**Europäisches Patentamt**

**European Patent Office**

**Office européen des brevets**

(11) **EP 4 295 160 B1**

(12) **EUROPEAN PATENT SPECIFICATION**

(45) Date of publication and mention of the grant of the patent:
**23.07.2025 Bulletin 2025/30**

(21) Application number: **22706061.3**

(22) Date of filing: **16.02.2022**

(51) International Patent Classification (IPC):
*G01N 33/68* *(2006.01)*

(52) Cooperative Patent Classification (CPC):
**G01N 33/6893;** G01N 2800/06

(86) International application number:
**PCT/FI2022/050100**

(87) International publication number:
**WO 2022/175597 (25.08.2022 Gazette 2022/34)**

(54) **USE OF GLYCOPROTEIN ACETYLS FOR DETERMINING THE RISK OF DEVELOPING A GALLBLADDER DISORDER**

VERWENDUNG VON GLYKOPROTEINACETYLE ZUR BESTIMMUNG DES RISIKOS ZUR ENTWICKLUNG VON EINER GALLENBLASENERKRANKUNG

UTILISATION D'ACÉTYLES DE GLYCOPROTÉINES POUR DÉTERMINER LE RISQUE DE DÉVELOPPER UNE MALADIE DE LA VÉSICULE BILIAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.02.2021 FI 20215175**

(43) Date of publication of application:
**27.12.2023 Bulletin 2023/52**

(73) Proprietor: **NIGHTINGALE HEALTH OYJ**
**00300 Helsinki (FI)**

(72) Inventors:
 • **JULKUNEN, Heli**
  **00300 Helsinki (FI)**
 • **WÜRTZ, Peter**
  **00300 Helsinki (FI)**

(74) Representative: **Papula Oy**
**P.O. Box 981**
**00101 Helsinki (FI)**

(56) References cited:
 • **LIU ANGEN: "Proteomic-based analysis for identification of potential serum biomarkers in gallbladder cancer", ONCOLOGY REPORTS, 17 June 2011 (2011-06-17), XP055913382, ISSN: 1021-335X, DOI: 10.3892/or.2011.1353**
 • **WATANABE MASAO ET AL: "Alpha-fetoprotein-producing carcinoma of the gallbladder", DIGESTIVE DISEASES AND SCIENCES, vol. 38, no. 3, 1993, pages 561 - 564, XP009535085, ISSN: 0163-2116**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Description

## TECHNICAL FIELD

The present disclosure relates generally to methods for determining whether a subject is at risk of developing a digestive system disease.

## BACKGROUND

Digestive system diseases include diseases related to the gastrointestinal tract and other organs of digestion, such as the pancreas, gallbladder and esophagus. Common digestive system disorders include, for instance, gastroesophageal reflux disease and inflammatory bowel diseases, such as Crohn's disease and ulcerative colitis. Some digestive diseases and conditions are acute, while others are chronic and long-lasting in their effects, with symptoms varying from mild to severe. These disorders are a source for impaired quality of life, comorbidity and mortality and they contribute to immense healthcare costs.

Due to the importance of the digestive system and its direct impact on daily life, identifying individuals at an elevated risk for future onset of these diseases and related symptoms may be important for prevention and early treatment approaches. Various blood biomarkers may be useful for predicting whether an individual person is at an elevated risk of developing a broad range digestive system disorders, such as malignant neoplasms of digestive organs, diseases of esophagus, stomach and duodenum, hernia, noninfective enteritis and colitis, diseases of intestines, diseases of peritoneum and retroperitoneum and disorders of gallbladder, biliary tract and pancreas. Biomarkers predictive of the onset of these disorders would help to enable more effective screening and better targeted early prevention. Such biomarkers may be measured from biological samples, for example from blood samples or related biological fluids.

Tan et al., Proteomic-based analysis for identification of potential serum biomarkers in gallbladder cancer, Oncology Reports 2011, DOI: 10.3892/or.2011.1353 describes protein spots significantly changed in patients with gallbladder cancer.

Watanabe et al., Alpha-fetoprotein-producing carcinoma of the gallbladder, Digestive Diseases and Sciences 1993, 38, 3, 561-564 describes an alpha-fetoprotein producing carcinoma of the gallbladder on which morphologic, immunohistochemical, and biochemical studies were performed.

## SUMMARY

A method for determining whether a subject is at risk of developing a digestive system disease is disclosed. The method comprises determining in a biological sample obtained from the subject a quantitative value of at least one biomarker of the following:

- albumin,
- ratio of docosahexaenoic acid to total fatty acids,
- ratio of linoleic acid to total fatty acids
- ratio of monounsaturated fatty acids and/or of oleic acid to total fatty acids,
- ratio of omega-3 fatty acids to total fatty acids,
- ratio of omega-6 fatty acids to total fatty acids,
- fatty acid degree of unsaturation,
- docosahexaenoic acid,
- linoleic acid,
- monounsaturated fatty acids and/or oleic acid,
- omega-3 fatty acids,
- omega-6 fatty acids,
- triglycerides in high-density lipoprotein (HDL),
- triglycerides in intermediate-density lipoprotein (IDL),
- triglycerides in low-density lipoprotein (LDL),
- triglycerides in very-low-density lipoprotein (VLDL),
- high-density lipoprotein (HDL) particle size,
- low-density lipoprotein (LDL) particle size,
- very-low-density lipoprotein (VLDL) particle size,
- acetate,
- citrate,
- creatinine,

- glutamine,
- glycine,
- isoleucine,
- phenylalanine,
- glycoprotein acetyls; and

comparing the quantitative value(s) of the at least one biomarker to a control sample or to a control value; wherein an increase or a decrease in the quantitative value(s) of at least one biomarker, when compared to the control sample or to the control value, is/are indicative of the subject having an increased risk of developing the digestive system disease; wherein the at least one biomarker comprises or is glycoprotein acetyls, wherein glycoprotein acetyls refers to a nuclear magnetic resonance spectroscopy signal that represents the abundance of circulating glycated proteins; and wherein the digestive system disease is cholelithiasis (ICD-10 diagnosis code K80); cholecystitis (ICD-10 diagnosis code K81); and/or other disease of gallbladder (ICD-10 diagnosis code K82).

## BRIEF DESCRIPTION OF THE DRAWINGS

The accompanying drawings, which are included to provide a further understanding of the embodiments and constitute a part of this specification, illustrate various embodiments. In the drawings:

**Figure 1a** shows the relation of baseline concentrations of 27 blood biomarkers to the future development of Any Digestive System Disease (defined as the combined endpoint of any ICD-10 diagnoses within C15-C28, K20-K67 and/or K80-K87; here termed "Any Digestive System Disease"), when the biomarker concentrations are analysed in absolute concentrations and in quintiles of biomarker concentrations. Results are based on plasma samples from approximately 115 000 generally healthy individuals from the UK Biobank.
**Figure 1b** shows the cumulative risk for "Any Digestive System Disease" during follow-up for the lowest, middle, and highest quintiles of the 27 blood biomarker concentrations.
**Figure 2a** shows the relation of the baseline concentrations of the 27 blood biomarkers to future development of 7 different categories of digestive system diseases (defined by ICD-10 subchapters), in the form of a heatmap. The results demonstrate that the 7 different digestive system disease subgroups all have highly similar associations with the 27 biomarkers measured by nuclear magnetic resonance (NMR) spectroscopy of plasma samples from generally healthy humans.
**Figure 2b** shows the consistency of the biomarker associations with the 7 different categories of digestive system diseases, defined by ICD-10 subchapters, in comparison to the direction of corresponding biomarker associations with "Any Digestive System Disease".
**Figure 3a** shows the relation of baseline biomarker levels to the future development of 34 specific digestive system diseases (defined by ICD-10 3-character diagnoses) in the form of a heatmap. The results demonstrate that the specific digestive system diseases defined by 3-character ICD-10 codes all have highly similar associations with a broad panel of biomarkers measured by NMR spectroscopy of plasma samples from generally healthy humans.
**Figure 3b** shows the consistency of the biomarker associations with specific digestive system diseases (defined by ICD-10 3-character diagnoses), in comparison to direction of the association with "Any Digestive System Disease".
**Figures 4a, 4b, 4c and 4d** show the relation of baseline biomarker levels to the future development of the 7 different digestive system disease categories (defined by ICD-10 subchapters), in the form of forestplots of the hazard ratios for incident disease onset.
**Figures 5a, 5b, 5c, 5d, 5e, 5f, 5g, 5h, 5i, 5j, 5k, 5l, 5m, 5n, 5o, 5p, and 5q** show the relation of baseline biomarker levels to the future development of 34 specific digestive system diseases (defined by ICD-10 3-character diagnoses), in the form of forestplots of the hazard ratios for incident disease onset.
**Figure 6** shows the relation of baseline concentrations of the 27 blood biomarkers to the risk of death from Any Digestive System Disease as compared to the risk of future hospitalisation and/or death from Any Digestive System Disease. The results demonstrate that the biomarkers may often be stronger predictors for fatal events than for hospitalisation.
**Figure 7** shows an example of the relation of multi-biomarker scores to the risk of "Any Digestive System Disease". Selected examples of multi-biomarker scores are shown to illustrate the improved prediction attained by multi-biomarker scores as compared to individual biomarkers.
**Figure 8a** shows an intended use case for a multi-biomarker score to predict the risk for developing malignant neoplasms of digestive organs among initially healthy humans.
**Figure 8b** shows that the prediction of the risk for developing malignant neoplasms of digestive organs works effectively for people with different demographics and risk factor profiles.

**Figure 9a** shows an intended use case for a multi-biomarker score to predict the risk for developing diseases of esophagus, stomach and duodenum among initially healthy humans.

**Figure 9b** shows that the prediction of the risk for developing diseases of esophagus, stomach and duodenum works effectively for people with different demographics and risk factor profiles.

**Figure 10a** shows an intended use case for a multi-biomarker score to predict the risk for developing hernia among initially healthy humans.

**Figure 10b** shows that the prediction of the risk for hernia works effectively for people with different demographics and risk factor profiles.

**Figure 11a** shows an intended use case for a multi-biomarker score to predict the risk for developing noninfective enteritis and colitis among initially healthy humans.

**Figure 11b** shows that the prediction of the risk for developing noninfective enteritis and colitis works effectively for people with different demographics and risk factor profiles.

**Figure 12a** shows an intended use case for a multi-biomarker score to predict the risk for developing other diseases of intestines among initially healthy humans.

**Figure 12b** shows that the prediction of the risk for developing other diseases of intestines works effectively for people with different demographics and risk factor profiles.

**Figure 13a** shows an intended use case for a multi-biomarker score to predict the risk for developing diseases of peritoneum and retroperitoneum among initially healthy humans.

**Figure 13b** shows that the prediction of the risk for developing diseases of peritoneum and retroperitoneum works effectively for people with different demographics and risk factor profiles.

**Figure 14a** shows an intended use case for a multi-biomarker score to predict the risk for developing disorders of gallbladder, biliary tract and pancreas among initially healthy humans.

**Figure 14b** shows that the prediction of the risk for developing disorders of gallbladder, biliary tract and pancreas works effectively for people with different demographics and risk factor profiles.

## DETAILED DESCRIPTION

A method for determining whether a subject is at risk of developing a digestive system disease is disclosed.

The method comprises determining in a biological sample obtained from the subject a quantitative value of at least one biomarker of the following:

- albumin,
- ratio of docosahexaenoic acid to total fatty acids,
- ratio of linoleic acid to total fatty acids,
- ratio of monounsaturated fatty acids and/or of oleic acid to total fatty acids,
- ratio of omega-3 fatty acids to total fatty acids,
- ratio of omega-6 fatty acids to total fatty acids,
- fatty acid degree of unsaturation,
- docosahexaenoic acid,
- linoleic acid,
- monounsaturated fatty acids and/or oleic acid,
- omega-3 fatty acids,
- omega-6 fatty acids,
- triglycerides in high-density lipoprotein (HDL),
- triglycerides in intermediate-density lipoprotein (IDL),
- triglycerides in low-density lipoprotein (LDL),
- triglycerides in very-low-density lipoprotein (VLDL),
- high-density lipoprotein (HDL) particle size,
- low-density lipoprotein (LDL) particle size,
- very-low-density lipoprotein (VLDL) particle size,
- acetate,
- citrate,
- creatinine,
- glutamine,
- glycine,
- isoleucine,
- phenylalanine,
- glycoprotein acetyls; and

and comparing the quantitative value(s) of the at least one biomarker to a control sample or to a control value; wherein an increase or a decrease in the quantitative value(s) of the at least one biomarker, when compared to the control sample or to the control value, is/are indicative of the subject having an increased risk of developing the digestive system disease.

Various blood biomarkers may be useful for predicting whether an individual person is at an elevated risk of developing a broad range of digestive system diseases. Such biomarkers may be measured from biological samples, for example from blood samples or related biological fluids.

Biomarkers predictive of digestive system diseases could help to enable more effective screening and better targeted preventative treatment.

In an embodiment, the method comprises determining a quantitative value of albumin.

The method comprises determining a quantitative value of glycoprotein acetyls.

In an embodiment, the method comprises determining a quantitative value of ratio of docosahexaenoic acid to total fatty acids.

In an embodiment, the method comprises determining a quantitative value of ratio of linoleic acid to total fatty acids.

In an embodiment, the method comprises determining a quantitative value of ratio of monounsaturated fatty acids and/or oleic acid to total fatty acids.

In an embodiment, the method comprises determining a quantitative value of ratio of omega-3 fatty acids to total fatty acids.

In an embodiment, the method comprises determining a quantitative value of ratio of omega-6 fatty acids to total fatty acids.

In an embodiment, the method comprises determining a quantitative value of fatty acid degree of unsaturation.

In an embodiment, the method comprises determining a quantitative value of docosahexaenoic acid.

In an embodiment, the method comprises determining a quantitative value of linoleic acid.

In an embodiment, the method comprises determining a quantitative value(s) of monounsaturated fatty acids and/or oleic acid.

In an embodiment, the method comprises determining a quantitative value of omega-3 fatty acids.

In an embodiment, the method comprises determining a quantitative value of omega-6 fatty acids.

In an embodiment, the method comprises determining a quantitative value of triglycerides in high-density lipoprotein (HDL) .

In an embodiment, the method comprises determining a quantitative value of triglycerides in intermediate-density lipoprotein (IDL).

In an embodiment, the method comprises determining a quantitative value of triglycerides in low-density lipoprotein (LDL) .

In an embodiment, the method comprises determining a quantitative value of triglycerides in very-low-density lipoprotein (VLDL).

In an embodiment, the method comprises determining a quantitative value of high-density lipoprotein (HDL) particle size.

In an embodiment, the method comprises determining a quantitative value of low-density lipoprotein (LDL) particle size.

In an embodiment, the method comprises determining a quantitative value of very-low-density lipoprotein (VLDL) particle size.

In an embodiment, the method comprises determining a quantitative value of acetate.

In an embodiment, the method comprises determining a quantitative value of citrate.

In an embodiment, the method comprises determining a quantitative value of creatinine.

In an embodiment, the method comprises determining a quantitative value of glutamine.

In an embodiment, the method comprises determining a quantitative value of glycine.

In an embodiment, the method comprises determining a quantitative value of isoleucine.

In an embodiment, the method comprises determining a quantitative value of phenylalanine.

The metabolic biomarker (s) described in this specification have been found to be significantly different, i.e. their quantitative values (such as amount and/or concentration) have been found to be significantly higher or lower, for subjects who later developed a digestive system disease. The biomarkers may be detected and quantified from blood, serum, or plasma, dry blood spots, or other suitable biological sample, and may be used to determine the risk of developing the digestive system disease, either alone or in combination with other biomarkers.

Furthermore, the biomarker(s) may significantly improve the possibility of identifying subjects at risk for a digestive system disease, even in combination with and/or when accounting for established risk factors that may currently be used for screening and risk prediction, such as age, sex, smoking status, use of alcohol, body mass index (BMI), physical activity, dietary factors such as low-fiber or high-fat diet, stress, use of certain medicines such as antacids, iron pills, strong pain medicines or laxatives, genetic risk and/or prior medical and/or family history of having digestive system diseases and/or other comorbidities. The biomarkers described in this specification, alone or as a risk score (such as a multi-biomarker score), hazard ratio, odds ratio, and/or predicted absolute or relative risk, or in combination with other risk factors (such as the risk factors described above) and tests, may improve prediction or even replace the need for other tests or measures. This may include improving prediction accuracy by complementing the predictive information from other risk factors, or by replacing the need for other analyses and tests, such as blood tests for complete blood count (CBC) and/or white blood cell count, blood tests for inflammatory protein biomarkers such as C-reactive protein (CRP), fecal calprotectin measurement, and/or examinations including, for example, colonoscopy, esophagogastroduodenoscopy, capsule endoscopy, endoscopic retrograde cholangiopancreatography (ERCP) and/or endoscopic ultrasound. The biomarkers or the risk score, hazard ratio, odds ratio, and/or predicted absolute or relative risk according to one or more embodiments described in this specification may thus allow for efficiently assessing the risk for future development of a digestive system disease, also in conditions in which other risk factor measures are not as feasible.

In an embodiment, the method is a method for determining whether the subject is at risk of dying from the digestive system disease. In other words, the method may be a method for determining whether the subject is at risk of a fatal form of the digestive system disease.

In an embodiment, the method is a method for determining whether the subject is at risk of developing or dying from the digestive system disease within (the following) three years.

The method may comprise determining in the biological sample quantitative values of a plurality of the biomarkers, such as two, three, four, five or more biomarkers. For example, the plurality of the biomarkers may comprise 2, 3, 4, 5, 6, 7, 8, 9, 10, 11, 12, 13, 14, 15, 16, 17, 18, 19, 20, 21, 22, 23, 24, 25, 26 or 27 (i.e. at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26) or all of the biomarkers. The term "plurality of the biomarkers" may thus, within this specification, be understood as referring to any number (above one) of the biomarkers. The term "plurality of the biomarkers" may thus be understood as referring to any number (above one) and/or combination or subset of the biomarkers described in this specification. Determining the plurality of the biomarkers may increase the accuracy of the prediction of whether the subject is at risk of developing the digestive system disease. In general, it may be that the higher the number of the biomarkers, the more accurate or predictive the method. However, even a single biomarker described in this specification may allow for or assist in determining whether the subject is at risk of developing the digestive system disease. The plurality of the biomarkers may be measured from the same biological sample or from separate biological samples and using the same analytical method or different analytical methods. In an embodiment, the plurality of biomarkers may be a panel of a plurality of biomarkers.

In the context of this specification, the wording "comparing the quantitative value(s) of the biomarker(s) to a control sample or to a control value(s)" may be understood, as a skilled person would, as referring to comparing the quantitative value or values of the biomarker or biomarkers, to a control sample or to a control value(s) either individually or as a plurality of biomarkers (e.g. when a risk score is calculated from the quantitative values of a plurality of biomarkers), depending e.g. on whether the quantitative value of a single (individual) biomarker or the quantitative values of a plurality of biomarkers are determined.

In an embodiment, the method may comprise determining in the biological sample obtained from the subject a quantitative value of the following biomarkers:

- albumin,
- ratio of docosahexaenoic acid to total fatty acids,
- ratio of linoleic acid to total fatty acids,
- ratio of monounsaturated fatty acids and/or of oleic acid to total fatty acids,
- ratio of omega-3 fatty acids to total fatty acids,
- ratio of omega-6 fatty acids to total fatty acids,
- fatty acid degree of unsaturation,
- docosahexaenoic acid,
- linoleic acid,
- monounsaturated fatty acids and/or oleic acid,
- omega-3 fatty acids,
- omega-6 fatty acids,
- triglycerides in high-density lipoprotein (HDL),
- triglycerides in intermediate-density lipoprotein (IDL),
- triglycerides in low-density lipoprotein ( LDL),
- triglycerides in very-low-density lipoprotein (VLDL),
- high-density lipoprotein (HDL) particle size,

- low-density lipoprotein (LDL) particle size,
- very-low-density lipoprotein (VLDL) particle size,
- acetate,
- citrate,
- creatinine,
- glutamine,
- glycine,
- isoleucine,
- phenylalanine,
- glycoprotein acetyls; and

comparing the quantitative value(s) of the biomarkers to a control sample or to a control value(s);
wherein an increase or a decrease in the quantitative value(s) of the biomarkers, when compared to the control sample or to the control value, is/are indicative of the subject having an increased risk of developing the digestive system disease.

The at least one biomarker comprises or is glycoprotein acetyls. The method may further comprise determining a quantitative value of at least one of the other biomarkers described in this specification.

The digestive system disease is cholelithiasis (K80); cholecystitis (K81); and/or other disease of gallbladder (K82).

The subject may be human. The human may be healthy or have an existing disease, such as an existing digestive system disease. Specifically, the human may have an already existing form of a digestive system disease, and the risk for developing more severe forms of said disease and/or of one or more other digestive system diseases may be determined and/or calculated. The subject may, additionally or alternatively, be an animal, such as a mammal, for example, a non-human primate, a dog, a cat, a horse, or a rodent.

In the context of this specification, the term "biomarker" may refer to a biomarker, for example a chemical or molecular marker, that may be found to be associated with a disease or a condition or the risk of having or developing thereof. It does not necessarily refer to a biomarker that would be statistically fully validated as having a specific effectiveness in a clinical setting. The biomarker may be a metabolite, a compound, a lipid, a protein, a moiety, a functional group, a composition, a combination of two or more metabolites and/or compounds, a (measurable or measured) quantity thereof, a ratio or other value derived thereof, or in principle any measurement reflecting a chemical and/or biological component that may be found associated with a disease or condition or the risk of having or developing thereof. The biomarkers and any combinations thereof, optionally in combination with further analyses and/or measures, may be used to measure a biological process indicative of the risk for developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or a disorder of gallbladder, biliary tract and/or pancreas.

The disorder or disease may refer to a category of digestive system diseases or to a specific disorder in this category. In the context of this specification, the term "a digestive system disease" may be understood as referring to diseases, disorders and/or conditions affecting the digestive system, including the gastrointestinal tract and/or other organs of digestion, such as the pancreas, gallbladder and/or esophagus. The disorder or disease may be acute or occasional, or a chronic condition, which in the context of this specification may be understood as persistent or otherwise long-lasting in its effects and/or a disease that comes with time. The signs and symptoms of digestive system diseases may vary from mild to severe or disabling, depending on factors such as age and/or overall health of the subject.

The biomarker associations may be similar for the different digestive system diseases. Therefore, the same individual biomarkers and combinations of biomarkers may be extended to also predict the risk for specific digestive system diseases. Examples of such specific digestive system diseases include malignant neoplasms of digestive organs, diseases of esophagus, stomach and/or duodenum, hernia, noninfective enteritis and/or colitis, other diseases of intestines, diseases of peritoneum and/or retroperitoneum and/or disorders of gallbladder, biliary tract and/or pancreas.

Digestive system diseases described in this specification may be classified as follows. "ICD-10" may be understood as referring to the International Statistical Classification of Diseases and Related Health Problems 10th Revision (ICD-10) - WHO Version for 2019. Similar diseases classified or diagnosed by other disease classification systems than ICD-10, such as ICD-9 or ICD-11, may also apply.

The term "Any Digestive System Disease" may be understood as referring to any disease, disorder or condition of the digestive system. Any Digestive System Disease may be understood as referring to any incident occurrence of ICD-10 diagnoses C15-C28, K20-K67 and/or K80-K87.

Digestive system disease Subgroups may be understood as referring to diseases and/or conditions classified within the ICD-10 subchapter diagnoses for digestive system diseases (C15-C26, K20-K31, K40-K46, K50-K52, K55-K64, K65-K68, K80-K87).

Specific digestive system diseases may be understood as referring to diseases and classified within the 3-character ICD-10 diagnoses for digestive system diseases (C15, C16, C18, C19, C25, K20, K21, K22, K25, K26, K29, K31, K42, K43, K44, K50, K51, K52, K55, K56, K57, K58, K60, K61, K62, K63, K65, K66, K80, K81, K82, K83, K85, K86).

In an embodiment, the digestive system disease is at least one of the following ICD-10 3-character diagnoses:

- K80: Cholelithiasis
- K81: Cholecystitis
- K82: Other diseases of gallbladder

In an embodiment, the digestive system disease may comprise or be death from a digestive system disease, such as a disease denoted by the ICD-10 codes listed above.

The method may further comprise determining whether the subject is at risk of developing the digestive system disease using a risk score, hazard ratio, odds ratio, and/or predicted absolute or relative risk calculated on the basis of the quantitative value(s) of the at least one biomarker or of the plurality of the biomarkers.

An increase or a decrease in the risk score, hazard ratio, and/or predicted absolute risk and/or relative risk may be indicative of the subject having an increased risk of developing the digestive system disease.

The risk score, hazard ratio, odds ratio and/or predicted absolute risk or relative risk may be calculated based on any plurality, combination or subset of biomarkers described in this specification.

The risk score, hazard ratio, odds ratio and/or predicted absolute risk or relative risk may be calculated e.g. as shown in the Examples below. For example, the plurality of biomarkers measured using a suitable method, for example with NMR spectroscopy, may be combined using regression algorithms and multivariate analyses and/or using machine learning analysis. Before regression analysis or machine learning, any missing values in the biomarkers may be imputed with the mean value of each biomarker for the dataset. A number of biomarkers, for example five, that may be considered most associated with the onset of the disease or condition may be selected for use in the prediction model. Other modelling approaches may be used to calculate a risk score and/or hazard ratio and/or predicted absolute risk or relative risk based on a combination or subset of individual biomarkers, i.e. a plurality of the biomarkers.

The risk score may be calculated e.g. as a weighted sum of individual biomarkers, i.e. a plurality of the biomarkers. The weighted sum may be e.g. in the form of a multi-biomarker score defined as $\sum_i [\![ \beta_i * c_i ]\!] + \beta_0$; where $i$ is the index of summation over individual biomarkers, $\beta_i$ is the weighted coefficient attributed to biomarker $i$, $c_i$ is the blood concentration of biomarker $i$, and $\beta_0$ is an intercept term.

For example, the risk score can be defined as: $\beta_1$*concentration(glycoprotein acetyls) + $\beta_2$* concentration(monounsaturated fatty acid ratio to total fatty acids) + $\beta_3$* concentration (albumin) + $\beta_0$, where $\beta_1$, $\beta_2$, $\beta_3$ are multipliers for each biomarker according to the association magnitude with risk of the digestive system disease and $\beta_0$ is an intercept term. As a skilled person will understand, the biomarkers mentioned in this example may be replaced by any other biomarker(s) described in this specification. In general, the more biomarkers are included in the risk score, the stronger the predictive performance may become. When additional biomarkers are included in the risk score, the $\beta i$ weights may change for all biomarkers according to the optimal combination for the prediction of the digestive system disease.

The risk score, hazard ratio, odds ratio, and/or predicted relative risk and/or absolute risk may be calculated on the basis of at least one further measure, for example a characteristic of the subject. Such characteristics may be determined prior to, simultaneously, or after the biological sample is obtained from the subject. As a skilled person will understand, some of the characteristics may be information collected e.g. using a questionnaire or clinical data collected earlier. Some of the characteristics may be determined (or may have been determined) by biochemical or clinical diagnostic measurements and/or medical diagnosis. Such characteristics could include, for example, one or more of age, height, weight, body mass index, race or ethnic group, smoking, and/or family history of digestive system diseases. Such characteristics could include, additionally or alternatively, age, sex, smoking status, use of alcohol, body mass index (BMI), physical activity, dietary factors such as low-fiber or high-fat diet, stress, use of certain medicines such as antacids, iron pills, strong pain medicines or laxatives, genetic risk and/or prior medical and/or family history of having digestive system diseases and/or other comorbidities.

The risk prediction for the digestive system disease guided based on one or more of the biomarkers can be used to guide preventative efforts, such as alcohol and smoking awareness, healthy diet, increased intake of fiber and water, physical activity and/or clinical screening frequency and/or pharmacological treatment decisions. For example, the information of the future risk for the digestive system disease can be used for guiding dietary interventions and/or treatment with, for instance, antacids, alginic acids, histamine H2-receptor antagonists and/or bismuth subsalicylate.

In the context of this specification, the term "albumin" may be understood as referring to serum albumin (often referred to as blood albumin). It is an albumin found in vertebrate blood. Albumin is a globular, water-soluble, unglycosylated serum protein of approximate molecular weight of 65,000 Daltons. The measurement of albumin using NMR is described e.g. in publications by Kettunen et al., 2012, Nature Genetics 44, 269-276; Soininen et al., 2015, Circulation: Cardiovascular Genetics 8, 212-206 (DOI: 10.1161/CIRCGENETICS.114.000216). Albumin may also be measured by various other methods, for example by clinical chemistry analyzers. Examples of such methods may include e.g. dye-binding methods such as bromocresol green and bromocresol purple.

In the context of this specification, the term "glycoprotein acetyls", "glycoprotein acetylation", or "GlycA" refers to a nuclear magnetic resonance spectroscopy (NMR) signal that represents the abundance of circulating glycated proteins, i.e. N-acetylated glycoproteins. Glycoprotein acetyls may include signals from a plurality of different glycoproteins, including e.g. alpha-1-acid glycoprotein, alpha-1 antitrypsin, haptoglobin, transferrin, and/or alpha-1 antichymotrypsin. In the scientific literature on cardiometabolic biomarkers, the terms "glycoprotein acetyls" or "GlycA" may commonly refer to the NMR signal of circulating glycated proteins (e.g. Ritchie et al, Cell Systems 2015 1(4):293-301; Connelly et al, J Transl Med. 2017;15(1):219). Glycoprotein acetyls and a method for measuring them is described e.g. in Kettunen et al., 2018, Circ Genom Precis Med. 11:e002234 and Soininen et al., 2009, Analyst 134, 1781-1785. There may be benefits of using the NMR signal of glycoprotein acetyls for risk prediction above measurement of the individual proteins contributing to the NMR signal, for instance better analytical accuracy and stability over time, as well as lower costs of the measurement and the possibility to measure the NMR signal simultaneously with many other biomarkers.

In the context of this specification, the term "omega-3 fatty acids" may refer to total omega-3 fatty acids, i.e. the total omega-3 fatty acid amounts and/or concentrations, i.e. the sum of different omega-3 fatty acids. Omega-3 fatty acids are polyunsaturated fatty acids. In omega-3 fatty acids, the last double bond in the fatty acid chain is the third bond counting from the methyl end. Docosahexaenoic acid is an example of an omega-3 fatty acid.

In the context of this specification, the term "omega-6 fatty acids" may refer to total omega-6 fatty acids, i.e. the total omega-6 fatty acid amounts and/or concentrations, i.e. the sum of the amounts and/or concentrations of different omega-6 fatty acids. Omega-6 fatty acids are polyunsaturated fatty acids. In omega-6 fatty acids, the last double bond in the fatty acid chain is the sixth bond counting from the methyl end.

In one embodiment, the omega-6 fatty acid may be linoleic acid. Linoleic acid (18:2$\omega$-6) is the most abundant type of omega-6 fatty acids, and may therefore be considered as a good approximation for total omega-6 fatty acids for risk prediction of the digestive system disease.

In the context of this specification, the term "monounsaturated fatty acids" (MUFAs) may refer to total monounsaturated fatty acids, i.e. the total MUFA amounts and/or concentrations. Monounsaturated fatty acids may include oleic acid, which is the most abundant monounsaturated fatty acid in human serum. Monounsaturated fatty acids have one double bond in their fatty acid chain. The monounsaturated fatty acids may include omega-9 and omega-7 fatty acids. Oleic acid (18:1$\omega$-9), palmitoleic acid (16:1$\omega$-7) and cis-vaccenic acid (18:1$\omega$-7) are examples of common monounsaturated fatty acids in human serum.

In one embodiment, the monounsaturated fatty acid may be oleic acid. Oleic acid is the most abundant monounsaturated fatty acid, and may therefore be considered as a good approximation for total monounsaturated fatty acids for risk prediction of the digestive system disease.

For all fatty acid measures, including omega-6, docosahexaenoic acid, linoleic acid and/or monounsaturated fatty acids, the fatty acid measures may include blood (or serum/plasma) free fatty acids, bound fatty acids and esterified fatty acids. Esterified fatty acids may, for example, be esterified to glycerol as in triglycerides, diglycerides, monoglycerides, or phosphoglycerides, or to cholesterol as in cholesterol esters.

In the context of this specification, the term "fatty acid degree of unsaturation" or "unsaturation" may be understood as referring to the number of double bonds in total fatty acids, for example the average number of double bonds in total fatty acids.

In the context of this specification, the term "HDL" refers to high-density lipoprotein.

In the context of this specification, the term "IDL" refers to intermediate-density lipoprotein.

In the context of this specification, the term "LDL" refers to low-density lipoprotein.

In the context of this specification, the term "VLDL" refers to very-low-density lipoprotein.

In the context of this specification, the phrase "very-low-density lipoprotein (VLDL) triglycerides", "intermediate-density lipoprotein (IDL) triglycerides", "low-density lipoprotein (LDL) triglycerides", "high-density lipoprotein (HDL) triglycerides", "triglycerides in VLDL (very-low-density lipoprotein)", "triglycerides in IDL (intermediate-density lipoprotein)", "triglycerides in LDL (low-density lipoprotein)", or "triglycerides in HDL (high-density lipoprotein)", may be understood as referring to total triglyceride concentration in said lipoprotein class or subfraction.

In the context of this specification, the phrase "high-density lipoprotein (HDL) particle size", "low-density lipoprotein (LDL) particle size", or "very-low-density lipoprotein (VLDL) particle size", may be understood as referring to the average diameter for the particles in said lipoprotein class or subfraction.

In the context of this specification, the term "acetate" may refer to the acetate molecule and/or acetic acid, for example in blood, plasma or serum or related biofluids.

In the context of this specification, the term "citrate" may refer to the citrate molecule and/or citric acid, for example in blood, plasma or serum or related biofluids.

I In the context of this specification, the term "creatinine" may refer to the creatinine molecule, for example in blood, plasma or serum or related biofluids.

In the context of this specification, the term "glutamine" may refer to the glutamine amino acid, for example in blood, plasma or serum or related biofluids.

In the context of this specification, the term "glycine" may refer to the glycine amino acid, for example in blood, plasma or serum or related biofluids.

In the context of this specification, the term "isoleucine" may refer to the isoleucine amino acid, for example in blood, plasma or serum or related biofluids.

In the context of this specification, the term "phenylalanine" may refer to the phenylalanine amino acid, for example in blood, plasma or serum or related biofluids.

In the context of this specification, the term "quantitative value" may refer to any quantitative value characterizing the amount and/or concentration of a biomarker. For example, it may be the amount or concentration of the biomarker in the biological sample, or it may be a signal derived from nuclear magnetic resonance spectroscopy (NMR) or other method suitable for detecting the biomarker in a quantitative manner. Such a signal may be indicative of or may correlate with the amount or concentration of the biomarker. It may also be a quantitative value calculated from one or more signals derived from NMR measurements or from other measurements. Quantitative values may, additionally or alternatively, be measured using a variety of techniques. Such methods may include mass spectrometry (MS), gas chromatography combined with MS, high performance liquid chromatography alone or combined with MS, immunoturbidimetric measurements, ultracentrifugation, ion mobility, enzymatic analyses, colorimetric or fluorometric analyses, immunoblot analysis, immunohistochemical methods (e.g. *in situ* methods based on antibody detection of metabolites), and immunoassays (e.g. ELISA). Examples of various methods are set out below. The method used to determine the quantitative value(s) in the subject may be the same method that is used to determine the quantitative value(s) in a control subject/control subjects or in a control sample/control samples.

The quantitative value, or the initial quantitative value, of the at least one biomarker, or the plurality of the biomarkers, may be measured using nuclear magnetic resonance (NMR) spectroscopy, for example $^1$H-NMR. The at least one additional biomarker, or the plurality of the additional biomarkers, may also be measured using NMR. NMR may provide a particularly efficient and fast way to measure biomarkers, including a large number of biomarkers simultaneously, and can provide quantitative values for them. NMR also typically requires very little sample pre-treatment or preparation. The biomarkers measured with NMR can effectively be measured for large amounts of samples using an assay for blood (serum or plasma) NMR metabolomics previously published by Soininen et al., 2015, Circulation: Cardiovascular Genetics 8, 212-206 (DOI: 10.1161/CIRCGENETICS.114.000216); Soininen et al., 2009, Analyst 134, 1781-1785; and Würtz et al., 2017, American Journal of Epidemiology 186 (9), 1084-1096 (DOI: 10.1093/aje/kwx016). This provides data on 250 biomarkers per sample as described in detail in the above scientific papers.

In an embodiment, the (initial) quantitative value of the at least one biomarker is/are measured using nuclear magnetic resonance spectroscopy.

However, quantitative values for various biomarkers described in this specification may also be performed by techniques other than NMR. For example, mass spectrometry (MS), enzymatic methods, antibody-based detection methods, or other biochemical or chemical methods may be contemplated, depending on the biomarker.

E.g. monounsaturated fatty acids, omega-3 fatty acids, and omega-6 fatty acids can be quantified (i.e. their quantitative values may be determined) by serum total fatty acid composition using gas chromatography (for example, as described in Jula et al., 2005, Arterioscler Thromb Vasc Biol 25, 2152-2159).

In the context of this specification, the term "sample" or "biological sample" may refer to any biological sample obtained from a subject or a group or population of subjects. The sample may be fresh, frozen, or dry.

The biological sample may comprise or be, for example, a blood sample, a plasma sample, a serum sample, or a fraction obtainable therefrom, or a sample derived therefrom. The biological sample may be, for example, a fasting blood sample, a fasting plasma sample, a fasting serum sample, or a fraction obtainable therefrom. However, the biological sample does not necessarily have to be a fasting sample. The blood sample may be a venous blood sample.

The blood sample may be a dry blood sample. The dry blood sample may be a dried whole blood sample, a dried plasma sample, a dried serum sample, or a dried sample derived therefrom.

The biological sample may be obtained from the subject prior to determining the quantitative value of the at least one biomarker. Taking a blood sample or a tissue sample of a subject or patient is a part of normal clinical practice. The collected blood or tissue sample can be prepared and serum or plasma can be separated using techniques well known to a skilled person. Methods for separating one or more fractions from biological samples, such as blood samples or tissue samples, are also available to a skilled person. The term "fraction" may, in the context of this specification, also refer to a portion or a component of the biological sample separated according to one or more physical properties, for instance solubility, hydrophilicity or hydrophobicity, density, or molecular size.

In the context of this specification, the term "control sample" may refer to a sample obtained from a subject and known not to suffer from the disease or condition or not being at risk of having or developing the disease or condition. The control sample may be matched. In an embodiment, the control sample may be a biological sample from a healthy individual or a generalized population of healthy individuals. The term "control value" may be understood as a value obtainable from the control sample and/or a quantitative value derivable therefrom. For example, it may be possible to calculate a threshold value from control samples and/or control values, above or below which the risk of developing the disease or condition is elevated. In other words, a value higher or lower (depending on the biomarker, risk score, hazard ratio, and/or predicted absolute risk or relative risk) than the threshold value may be indicative of the subject having an increased risk of developing the disease or condition.

An increase or a decrease in the quantitative value(s) of the at least one biomarker, or the plurality of the biomarkers, when compared to the control sample or to the control value, may be indicative of the subject having an increased risk of having or developing the disease or condition. Whether an increase or a decrease is indicative of the subject having an increased risk of developing the disease or condition, may depend on the biomarker.

A 1.2-fold, 1.5-fold, or for example 2-fold, or 3-fold, increase or a decrease in the quantitative value(s) of the at least one biomarker (or in an individual biomarker of the plurality of the biomarkers) when compared to the control sample or to the control value, may be indicative of the subject having an increased risk of developing the disease or condition.

In an embodiment, a decrease in the quantitative value of albumin, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, an increase in the quantitative value of glycoprotein acetyls, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a decrease in the quantitative value of ratio of docosahexaenoic acid to total fatty acids, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a decrease in the quantitative value of ratio of linoleic acid to total fatty acids, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, an increase in the quantitative value of ratio of monounsaturated fatty acids and/or oleic acid to total fatty acids, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a decrease in the quantitative value of ratio of omega-3 fatty acids to total fatty acids, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a decrease in the quantitative value of ratio of omega-6 fatty acids to total fatty acids, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a decrease in the quantitative value of fatty acid degree of unsaturation, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a decrease in the quantitative value of docosahexaenoic acid, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a decrease in the quantitative value of linoleic acid, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, an increase in the quantitative value of monounsaturated fatty acids and/or oleic acid, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a decrease in the quantitative value of omega-3 fatty acids, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a decrease in the quantitative value of omega-6 fatty acids, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, an increase in the quantitative value of triglycerides in high-density lipoprotein (HDL), when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, an increase in the quantitative value of triglycerides in intermediate-density lipoprotein (IDL), when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, an increase in the quantitative value of triglycerides in low-density lipoprotein (LDL), when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, an increase in the quantitative value of triglycerides in very-low-density lipoprotein (VLDL), when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a decrease in the quantitative value of high-density lipoprotein (HDL) particle size, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a decrease in the quantitative value of low-density lipoprotein (LDL) particle size, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, an increase in the quantitative value of very-low-density lipoprotein (VLDL) particle size, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a decrease in the quantitative value of acetate, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a decrease in the quantitative value of citrate, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, an increase in the quantitative value of creatinine, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a decrease in the quantitative value of glutamine, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a decrease in the quantitative value of glycine, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, an increase in the quantitative value of isoleucine, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a increase in the quantitative value of phenylalanine, when compared to the control sample or to the control value, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a risk score defined as $\beta_0 + \beta_1 *$ concentration (glycoprotein acetyls) + $\beta_2 *$ concentration (albumin), where $\beta_0$ is an intercept term, $\beta_1$ is the weighted coefficient attributed to the concentration of glycoprotein acetyls, and $\beta_2$ is the weighted coefficient attributed to the concentration of albumin, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas.

In an embodiment, a risk score defined as $\beta_0 + \beta_1 *$ concentration (glycoprotein acetyls) + $\beta_2 *$ concentration (fatty acid measure), where $\beta_0$ is an intercept term, $\beta_1$ is the weighted coefficient attributed to the concentration of glycoprotein acetyls, $\beta_2$ is the weighted coefficient attributed to the fatty acid measure, may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas. The fatty acid measure may be one or more of the following fatty acids or their ratio to total fatty acids: docosahexaenoic acid, linoleic acid, omega-3 fatty acids, omega-6 fatty acids, monounsaturated fatty acids and/or fatty acid degree of unsaturation.

In an embodiment, a risk score defined as $\beta_0 + \beta_1 *$ concentration (glycoprotein acetyls) + $\beta_2 *$ concentration (albumin) + $\beta_3 *$ concentration (fatty acid measure), where $\beta_0$ is an intercept term, $\beta_1$ is the weighted coefficient attributed to the concentration of glycoprotein acetyls, $\beta_2$ is the weighted coefficient attributed to the concentration of albumin, and $\beta_3$ is the weighted coefficient attributed to the concentration of the fatty acid measure may be indicative of the subject having an increased risk of developing the digestive system disease, such as a malignant neoplasm of digestive organs, a disease of esophagus, stomach and/or duodenum, hernia, noninfective enteritis and/or colitis, other disease of intestines, a disease of peritoneum and/or retroperitoneum and/or disorder of gallbladder, biliary tract and/or pancreas. The fatty acid measure may be one or more of the following fatty acids or their ratio to total fatty acids: docosahexaenoic acid, linoleic acid, omega-3 fatty acids, omega-6 fatty acids, monounsaturated fatty acids and/or fatty acid degree of unsaturation.

The term "combination" may, at least in some embodiments, be understood such that the method comprises using a risk score, hazard ratio, odds ratio, and/or predicted absolute risk or relative risk calculated on the basis of the quantitative value(s) of the biomarkers. For example, if quantitative values of both glycoprotein acetyls and albumin are determined, the quantitative values of both biomarkers may be compared to the control sample or the control value separately, or a risk score, hazard ratio, odds ratio, and/or predicted absolute risk or relative risk calculated on the basis of the quantitative value(s) of both the biomarkers, and the risk score, hazard ratio, odds ratio, and/or predicted absolute risk or relative risk may be compared to the control sample or the control value.

In an embodiment, the method may comprise determining in the biological sample obtained from the subject a quantitative value of the following biomarkers:

- glycoprotein acetyls;
- albumin; and

comparing the quantitative value(s) of the biomarkers and/or a combination thereof to a control sample or to a control value(s);

wherein an increase or a decrease in the quantitative value(s) of the biomarkers and/or the combination thereof, when compared to the control sample or to the control value, is/are indicative of the subject having an increased risk of developing the digestive system disease. An increase in the quantitative value of glycoprotein acetyls and a decrease in the quantitative value of albumin, when compared to the control sample or to the control value, may be indicative of the subject having an increased risk of developing the digestive system disease.

In an embodiment, the method may comprise determining in the biological sample obtained from the subject a quantitative value of the following biomarkers:

- glycoprotein acetyls,
- at least one fatty acid measure(s) of the following fatty acids or their ratio to total fatty acids: docosahexaenoic acid, linoleic acid, omega-3 fatty acids, omega-6 fatty acids, monounsaturated fatty acids and/or fatty acid degree of unsaturation; and

comparing the quantitative value(s) of the biomarkers and/or a combination thereof to a control sample or to a control value(s);

wherein an increase or a decrease in the quantitative value(s) of the biomarkers and/or the combination thereof, when compared to the control sample or to the control value, is/are indicative of the subject having an increased risk of developing the digestive system disease. An increase in the quantitative value of glycoprotein acetyls and a decrease in the quantitative value of docosahexaenoic and/or linoleic acid and/or omega-3 fatty acids and/or omega-6 fatty acids and/or fatty acid degree of unsaturation and/or their ratio to total fatty acids, and/or an increase in the quantitative value of monounsaturated fatty acids and/or their ratio to total fatty acids, when compared to the control sample or to the control value, may be indicative of the subject having an increased risk of developing the digestive system disease.

In an embodiment, the method may comprise determining in the biological sample obtained from the subject a quantitative value of the following biomarkers:

- albumin,
- at least one fatty acid measure(s) of the following fatty acids or their ratio to total fatty acids: docosahexaenoic acid, linoleic acid, omega-3 fatty acids, omega-6 fatty acids, monounsaturated fatty acids and/or fatty acid degree of unsaturation; and

comparing the quantitative value(s) of the biomarkers and/or a combination thereof to a control sample or to a control value(s);

wherein an increase or a decrease in the quantitative value(s) of the biomarkers and/or the combination thereof, when compared to the control sample or to the control value, is/are indicative of the subject having an increased risk of developing the digestive system disease. A decrease in the quantitative value of albumin and a decrease in the quantitative value of docosahexaenoic and/or linoleic acid and/or omega-3 fatty acids and/or omega-6 fatty acids and/or fatty acid degree of unsaturation and/or their ratio to total fatty acids, and/or an increase in the quantitative value of monounsaturated fatty acids and/or their ratio to total fatty acids, when compared to the control sample or to the control value, may be indicative of the subject having an increased risk of developing the digestive system disease.

In an embodiment, the method may comprise determining in the biological sample obtained from the subject a quantitative value of the following biomarkers:

- glycoprotein acetyls,
- albumin,
- at least one fatty acid measure(s) of the following fatty acids or their ratio to total fatty acids: docosahexaenoic acid, linoleic acid, omega-3 fatty acids, omega-6 fatty acids, monounsaturated fatty acids and/or fatty acid degree of unsaturation; and

comparing the quantitative value(s) of the biomarkers and/or a combination thereof to a control sample or to a control value (s) ;
wherein an increase or a decrease in the quantitative value(s) of the biomarkers and/or the combination thereof, when compared to the control sample or to the control value, is/are indicative of the subject having an increased risk of developing the digestive system disease. An increase in the quantitative value of glycoprotein acetyls, a decrease in the quantitative value of albumin and a decrease in the quantitative value of docosahexaenoic and/or linoleic acid and/or omega-3 fatty acids and/or omega-6 fatty acids and/or fatty acid degree of unsaturation and/or their ratio to total fatty acids, and/or an increase in the quantitative value of monounsaturated fatty acids and/or their ratio to total fatty acids, when compared to the control sample or to the control value, may be indicative of the subject having an increased risk of developing the digestive system disease.

## EXAMPLES

Reference will now be made in detail to various embodiments, an example of which is illustrated in the accompanying drawings. The description below discloses some embodiments in such a detail that a person skilled in the art is able to utilize the embodiments based on the disclosure. Not all steps or features of the embodiments are discussed in detail, as many of the steps or features will be obvious for the person skilled in the art based on this specification.

**Abbreviations used in the Figures:**

DHA %: Ratio of docosahexaenoic acid to total fatty acids
LA%: Ratio of linoleic acid to total fatty acids
MUFA %: Ratio of monounsaturated fatty acids to total fatty acids
Omega-3 %: Ratio of omega-3 fatty acids to total fatty acids
Omega-6 %: Ratio of omega-6 fatty acids to total fatty acids
DHA: Docosahexaenoic acid
LA: Linoleic acid
MUFA: Monounsaturated fatty acids
Omega-6: Omega-6 fatty acids
Omega-3: Omega-3 fatty acids
Unsaturation: Fatty acid degree of unsaturation
HDL: High-density lipoprotein
LDL: Low-density lipoprotein
IDL: Intermediate-density lipoprotein
VLDL: Very-low-density lipoprotein
HDL-TG: Triglycerides in high-density lipoprotein (HDL)
LDL-TG: Triglycerides in low-density lipoprotein (HDL)
IDL-TG: Triglycerides in intermediate-density lipoprotein (IDL)
VLDL-TG: Triglycerides in very-low-density lipoprotein (VLDL)
CI: confidence interval
SD: standard deviation
BMI: Body mass index

## EXAMPLE 1

Biomarker measures quantified by nuclear magnetic resonance (NMR) were investigated as to whether they could be predictive of a digestive system disease, such as malignant neoplasms of digestive organs, diseases of esophagus, stomach and/or duodenum, hernia, noninfective enteritis and/or colitis, other diseases of intestines, diseases of peritoneum and/or retroperitoneum and/or disorders of gallbladder, biliary tract and/or pancreas. All analyses were conducted based on the UK Biobank, with approximately 115 000 study participants with blood biomarker data from NMR spectroscopy available.

## Study population

Details of the design of the UK Biobank have been reported by Sudlow et al 2015, PLoS Med. 2015;12(3):e1001779. Briefly, UK Biobank recruited 502 639 participants aged 37-73 years in 22 assessment centres across the UK. All participants provided written informed consent and ethical approval was obtained from the North West Multi-Center Research Ethics Committee. Blood samples were drawn at baseline between 2007 and 2010. No selection criteria were applied to the sampling.

## Biomarker profiling

From the entire UK Biobank population, a random subset of baseline plasma samples from 118 466 individuals were measured using the Nightingale NMR biomarker platform (Nightingale Health Ltd, Finland). This blood analysis method provides simultaneous quantification of many blood biomarkers, including lipoprotein lipids, circulating fatty acids, and various low-molecular weight metabolites including amino acids, ketone bodies and gluconeogenesis-related metabolites in molar concentration units. Technical details and epidemiological applications have been reviewed (Soininen et al 2015, Circ Cardiovasc Genet; 2015;8:192-206; Würtz et al 2017, Am J Epidemiol 2017;186:1084-1096). Values outside four interquartile ranges from median were considered as outliers and excluded.

## Epidemiological analyses of biomarker relations with the risk of a digestive system disease

The blood biomarker associations with the risk for a digestive system disease were conducted based on UK Biobank data. Analyses focused on the relation of the biomarkers to the occurrence of a digestive system disease after the blood samples were collected, to determine if the individual biomarkers associate with the risk for future development of a digestive system disease. Examples using multi-biomarker scores, in the form weighted sums of biomarkers, were also explored to see if they could be predictive even more strongly than each individual biomarker.

Information on the disease events occurring after the blood samplings for all study participants were recorded from UK Hospital Episode Statistics data and death registries. All analyses are based on first occurrence of diagnosis, so that individuals with recorded diagnosis of the given disease prior to blood sampling were omitted from the statistical analyses. A composite endpoint of Any Digestive System Disease was defined based on any incident occurrence of ICD-10 diagnoses C15-C28, K20-K67 and/or K80-K87. More refined subtypes of the digestive system diseases were defined according to the ICD-10 diagnoses listed in Table 1.

The registry-based follow-up was from blood sampling in 2007-2010 through to 2020 (approximately 1 100 000 person-years). Specific diseases which had <150 disease events recorded during follow-up were left out of scope.

For biomarker association testing, Cox proportional-hazard regression models adjusted for age, sex, and UK Biobank assessment centre were used. Results were plotted in magnitudes per standard deviation of each biomarker measure to allow direct comparison of association magnitudes.

## Summary of results

Baseline characteristics of the study population for biomarker analyses vs future risk of a digestive system disease are shown in Table 1. The number of incident disease events occurring after the blood sampling is listed for all the conditions analysed.

Table 1: Clinical characteristics of study participants and the number of incident disease events analysed.

| | |
|---|---|
| Total number of individuals with blood samples analysed | 118 456 |
| Study setting | Population sample of study volunteers from the UK |
| Percentage of women | 54.1% |
| Age range (years) | 39 – 71 |
| Median age (years) | 58 |
| Median BMI (kg/m2) | 26.8 |
| Follow-up time for disease events after blood sampling | 10-14 years |
| Diseases with similar biomarker relations | Number of individuals who developed the specified disease after the blood sampling |
| Any Digestive System Disease: any occurrence of C15-C28, K20-K67 and/or K80-K87 ICD-10 codes | 27 219 |
| Digestive System Disease Subgroups (defined by ICD-10 subchapters) | |

| | |
|---|---|
| C15-C26: Malignant neoplasms of digestive organs | 2 512 |
| K20-K31: Diseases of esophagus, stomach and duodenum | 15 651 |
| K40-K46: Hernia | 11 511 |
| K50-K52: Noninfective enteritis and colitis | 3 664 |
| K55-K64: Other diseases of intestines | 20 562 |
| K65-K68: Diseases of peritoneum and retroperitoneum | 1 643 |
| K80-K87: Disorders of gallbladder, biliary tract and pancreas | 4 556 |
| **Specific Digestive System Diseases(defined by 3-character ICD-10 codes)** | |
| C15: Malignant neoplasm of esophagus | 324 |
| C16: Malignant neoplasm of stomach | 243 |
| C18: Malignant neoplasm of colon | 1 065 |
| C19: Malignant neoplasm of rectosigmoid junction | 297 |
| C25: Malignant neoplasm of pancreas | 338 |
| K20: Esophagitis | 2 200 |
| K21: Gastro-esophageal reflux disease | 8 692 |
| K22: Other diseases of esophagus | 3 119 |
| K25: Gastric ulcer | 1 294 |
| K26: Duodenal ulcer | 682 |
| K29: Gastritis and duodenitis | 7 990 |
| K31: Other diseases of stomach and duodenum | 3 209 |
| K42: Umbilical hernia | 935 |
| K43: Ventral hernia | 946 |
| K44: Diaphragmatic hernia | 7 763 |
| K50: Crohn's disease [regional enteritis] | 309 |
| K51: Ulcerative colitis | 621 |
| K52: Other and unspecified noninfective gastroenteritis and colitis | 3 170 |
| K55: Vascular disorders of intestine | 555 |

| | |
|---|---|
| K56: Paralytic ileus and intestinal obstruction without hernia | 1 526 |
| K57: Diverticular disease of intestine | 10 773 |
| K58: Irritable bowel syndrome | 1 989 |
| K60: Fissure and fistula of anal and rectal regions | 508 |
| K61: Abscess of anal and rectal regions | 203 |
| K62: Other diseases of anus and rectum | 5 118 |
| K63: Other diseases of intestine | 6 062 |
| K65: Peritonitis | 287 |
| K66: Other disorders of peritoneum | 1 416 |
| K80: Cholelithiasis | 3 389 |
| K81: Cholecystitis | 640 |
| K82: Other diseases of gallbladder | 541 |
| K83: Other diseases of biliary tract | 824 |
| K85: Acute pancreatitis | 520 |
| K86: Other diseases of pancreas | 510 |

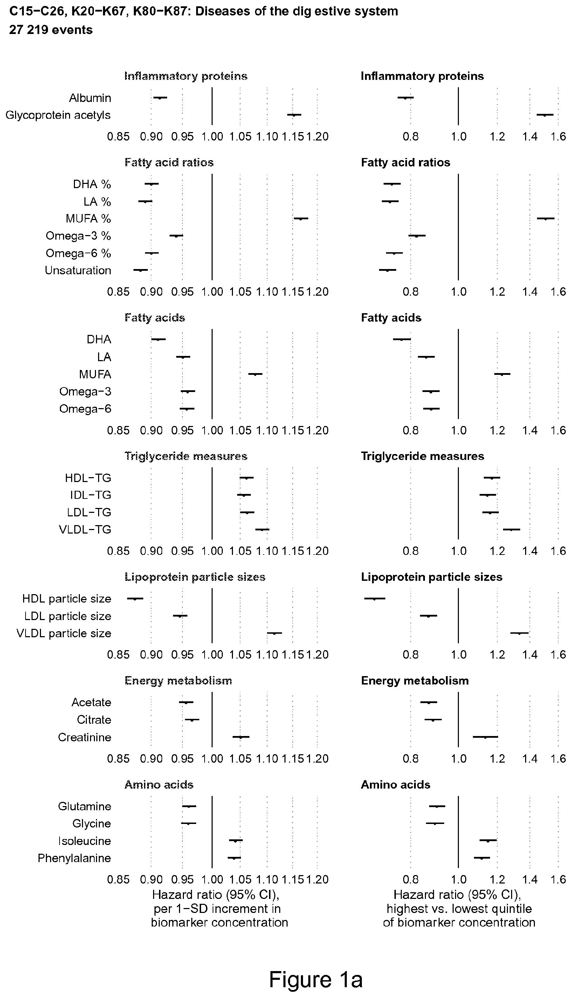

**Figure 1a** shows the hazard ratios for the 27 blood biomarkers with the future risk of Any Digestive System Disease (ICD-10 codes C15-C28, K20-K67 and/or K80-K87). The left-hand side of the figure shows the hazard ratios when the biomarkers are analysed in absolute concentrations, scaled to standard deviations of the study population. The right-hand side shows the corresponding hazard ratios when individuals in the highest quintile of the biomarker concentration are compared to those in the lowest quintile. The results are based on statistical analyses of over 115 000 individuals from the UK Biobank, out of whom 27 219 developed a digestive system disease (defined as diagnoses C15-C28, K20-K67 and/or K80-K87 in the hospital registries, or in the death records) during approximately 10 years of follow-up. The analyses were adjusted for age, sex, and UK Biobank assessment centre in Cox proportional-hazard regression models. P-values were P<0.0001 (corresponding to multiple testing correction) for all associations. These results demonstrate that the 27 individual biomarkers are predictive of the risk for a digestive system disease in general population settings.

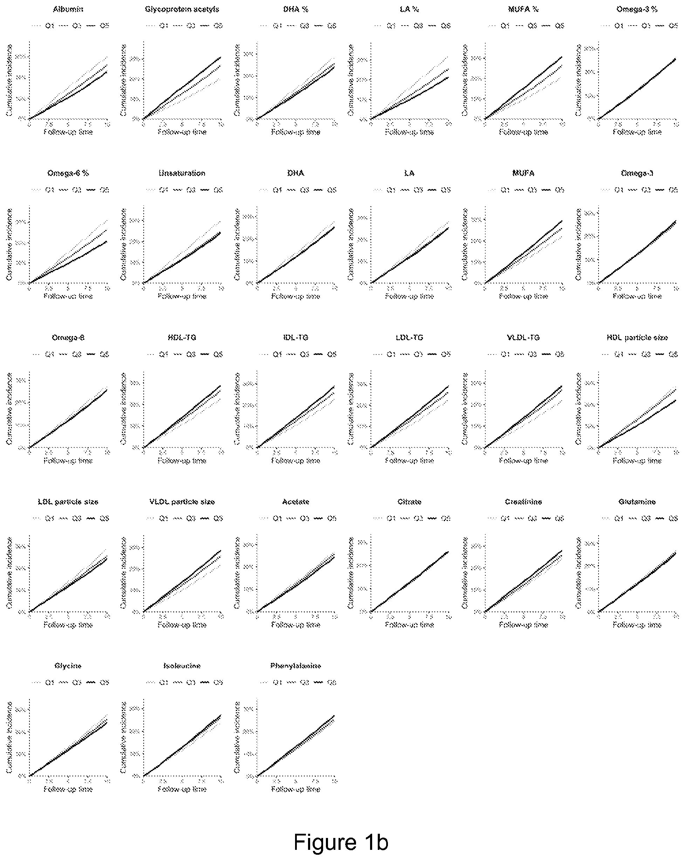

**Figure 1b** shows the Kaplan-Meier plots of the cumulative risk for a digestive system disease for each of the 27 blood biomarkers according to the lowest, middle, and highest quintiles of biomarker concentrations. The results are based on statistical analyses of over 115 000 individuals from the UK Biobank, out of whom 27 219 developed a digestive system disease. These results further demonstrate that the 27 individual biomarkers are predictive of the risk for a digestive system disease in general population settings.

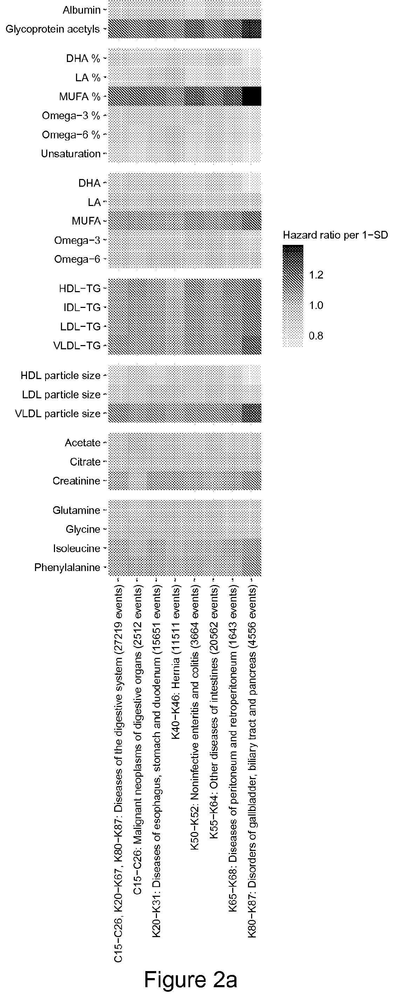

**Figure 2a** shows the hazard ratios for the 27 blood biomarkers for the future onset of 7 subgroups of digestive system diseases, defined by ICD-10 subchapters. The results illustrate that the pattern of biomarker associations is highly consistent for the 7 different subtypes of digestive system diseases.

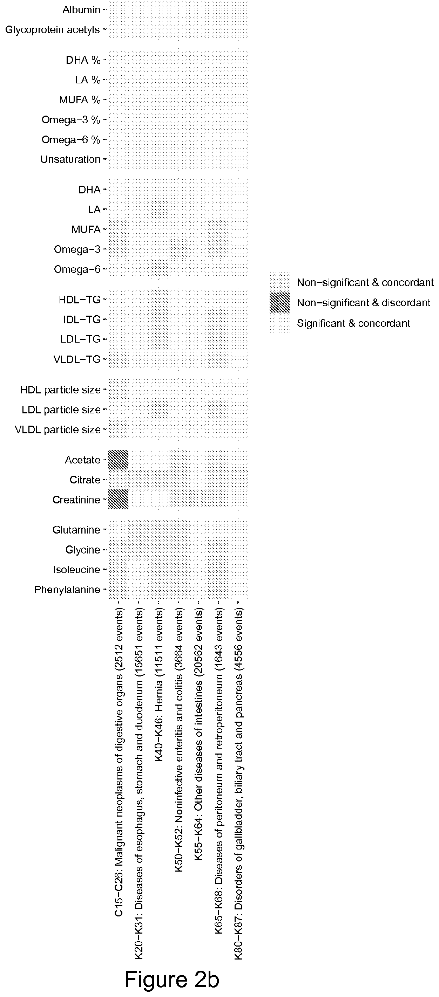

**Figure 2b** shows the consistency of the biomarker associations with the 7 digestive system disease subgroups (defined by ICD-10 subchapters) compared to the "Any Digestive System Disease" definition. The biomarker associations were all in the same direction of association as for "Any Digestive System Disease" or not statistically significant in the discordant direction. Any biomarker combination that strongly predicts "Any Digestive System Disease" will therefore also be predictive of all the listed digestive system disease subgroups.

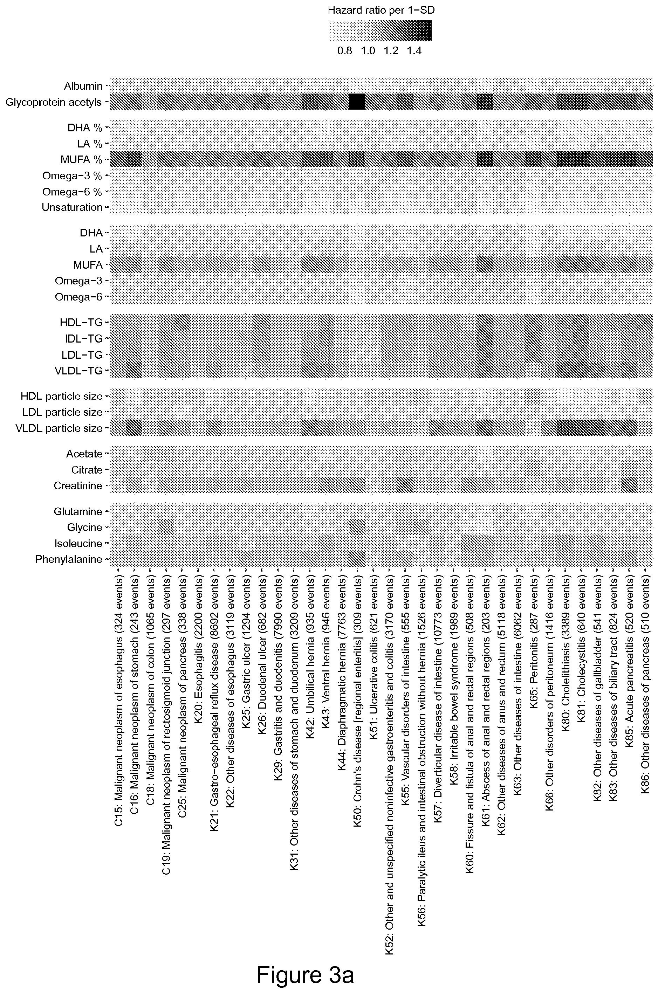

**Figure 3a** shows the hazard ratios for the 27 blood biomarkers for future onset of 34 specific digestive system diseases, defined by 3-character ICD-10 diagnosis codes. The results illustrate that the pattern of biomarker associations is highly consistent for all the 34 specific diseases.

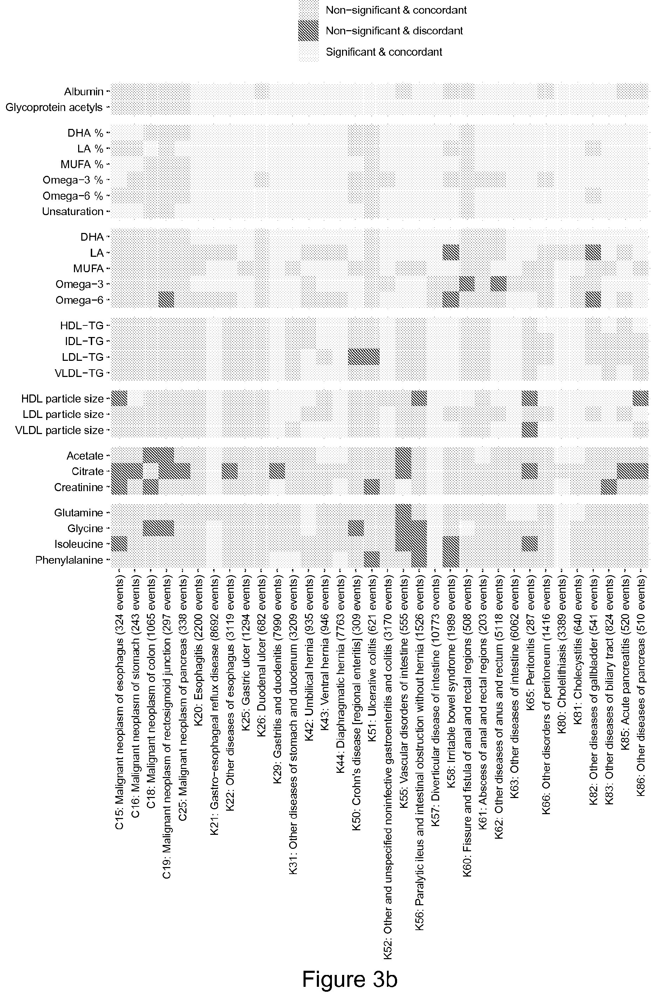

**Figure 3b** shows the consistency of the biomarker associations with the 34 specific digestive system diseases (defined by 3-character ICD-10 diagnosis codes) compared to the "Any Digestive System Disease" definition. The biomarker associations are all in the same direction of association as for "Any Digestive System Disease" or not statistically significant in the discordant direction. Any biomarker combination that strongly predicts "Any Digestive System Disease" will therefore also be predictive of all the listed specific digestive system diseases.

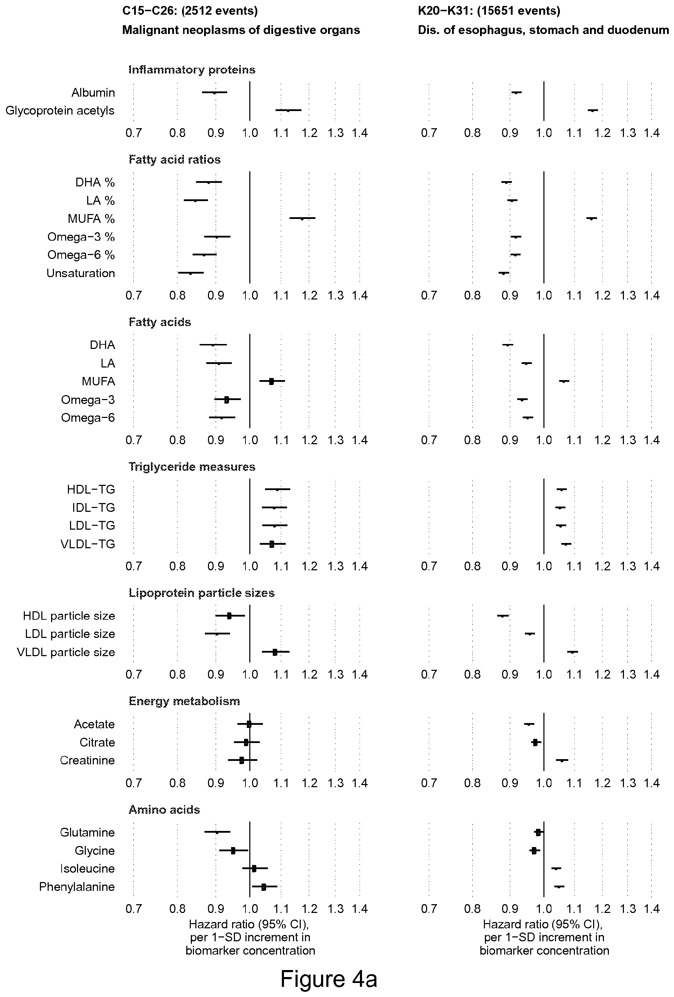

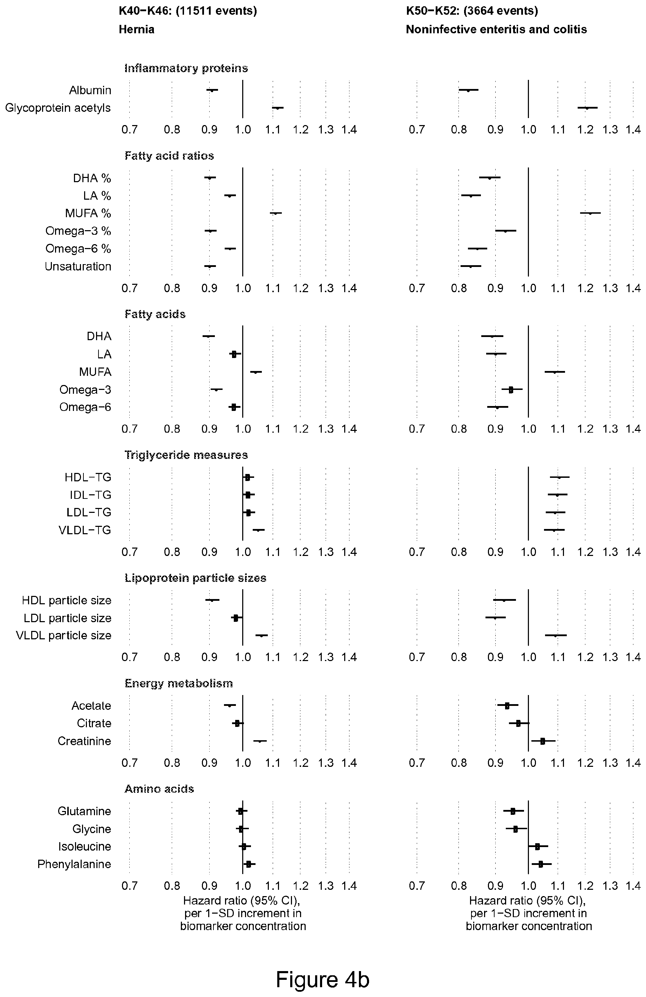

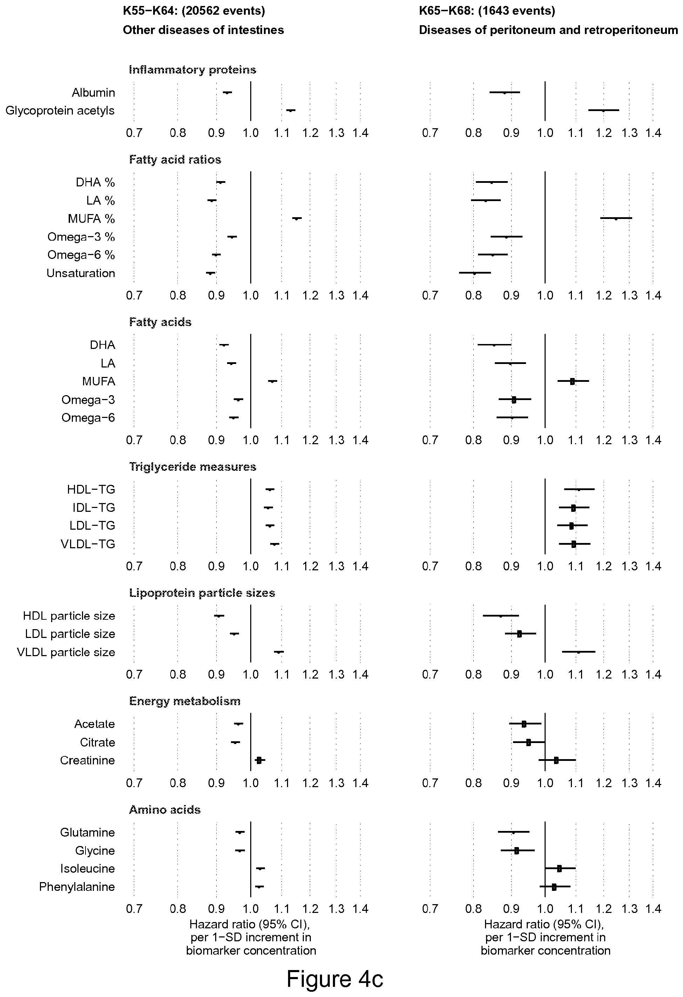

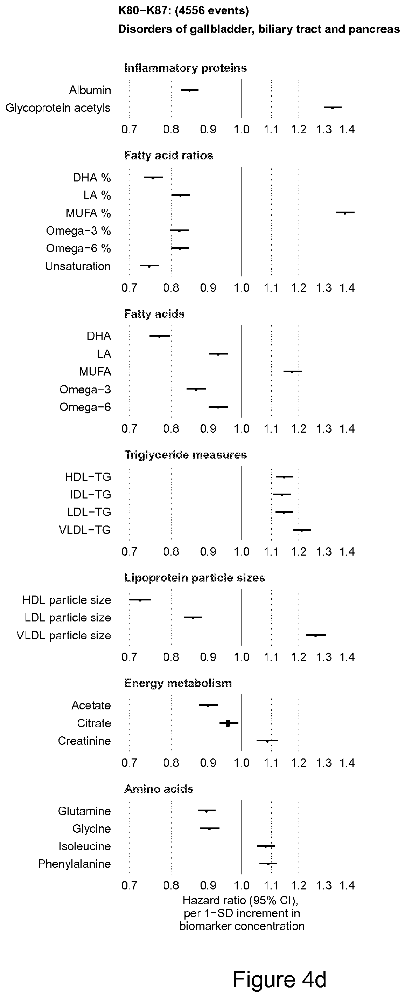

**Figures 4a-d** show the hazard ratios for the 27 blood biomarkers with future onset of each of the 7 digestive system disease subgroups (defined by ICD-10 subchapters) studied here. The hazard ratios are shown in absolute concentrations, scaled to the standard deviation of each biomarker. The results are based on statistical analyses of over 115 000 individuals from the UK Biobank; the number of individuals who developed the disorder during approximately 10 years of follow-up is indicated on the top of each plot. Filled circles denote that the P-value for association was P<0.0001 (corresponding to multiple testing correction), and open circles denote that the P-value for association was P≥0.0001. The analyses were adjusted for age, sex, and UK Biobank assessment centre using Cox proportional-hazard regression models.

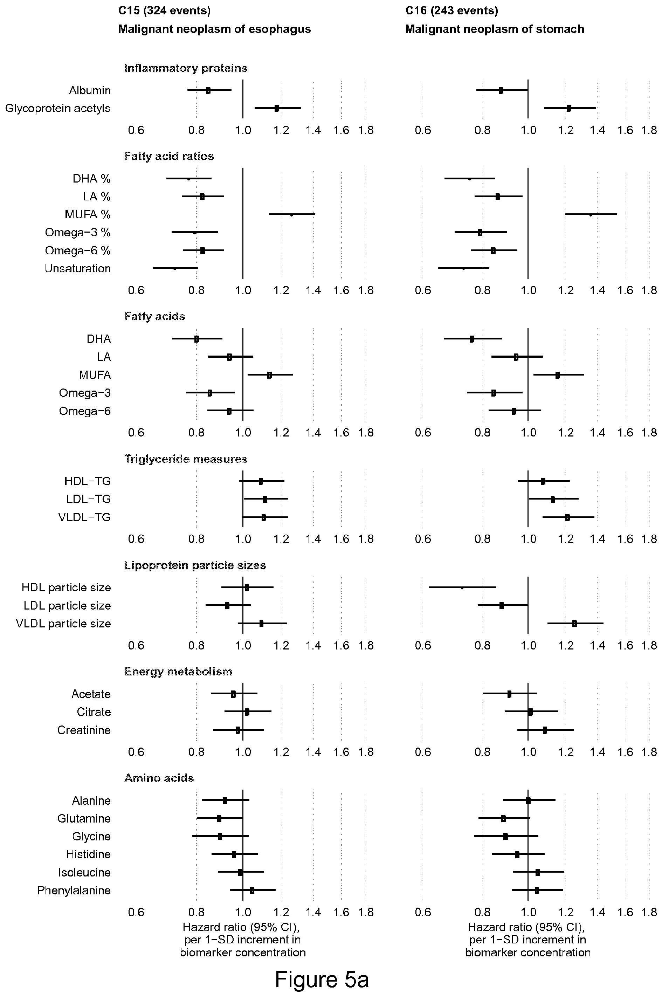

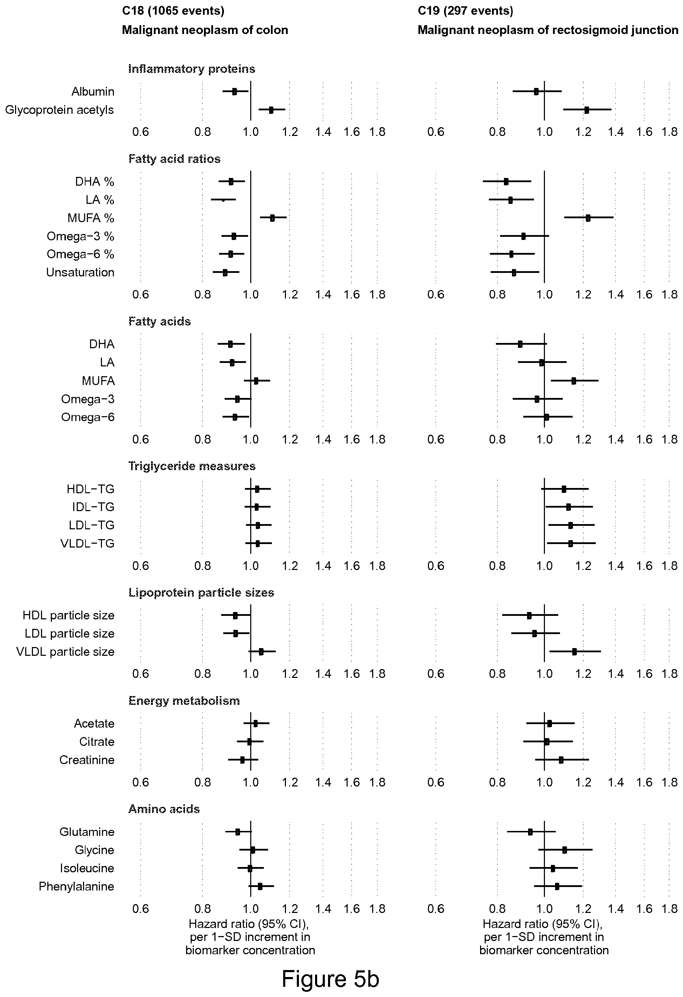

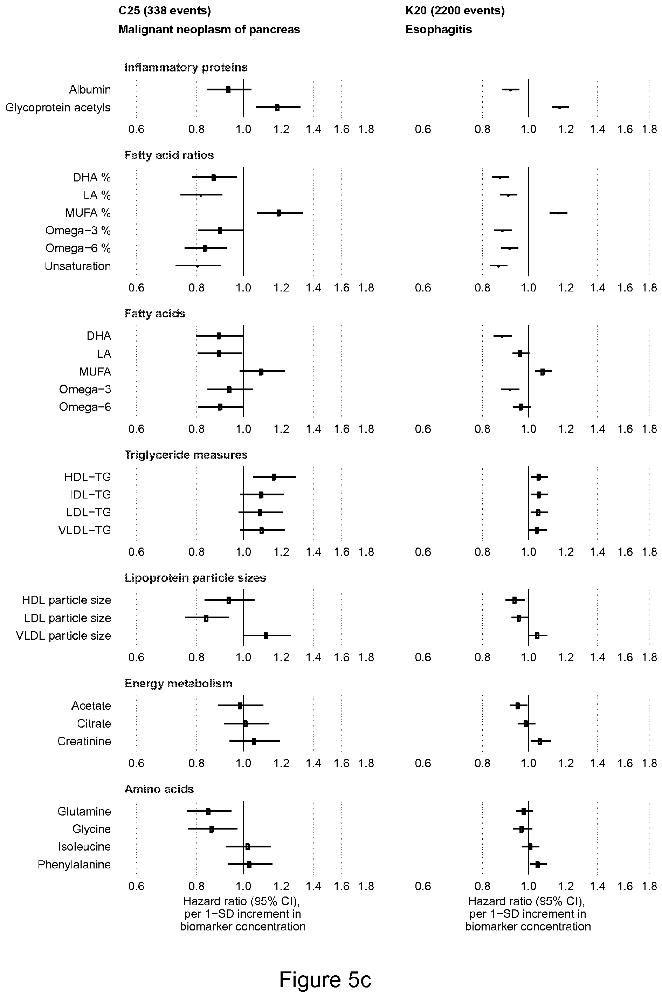

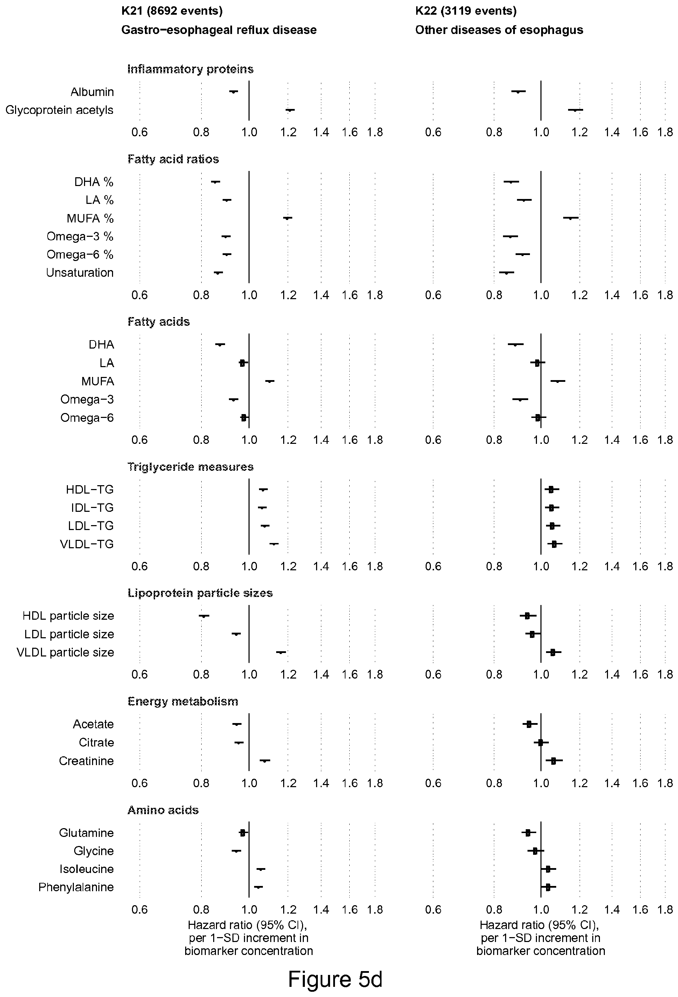

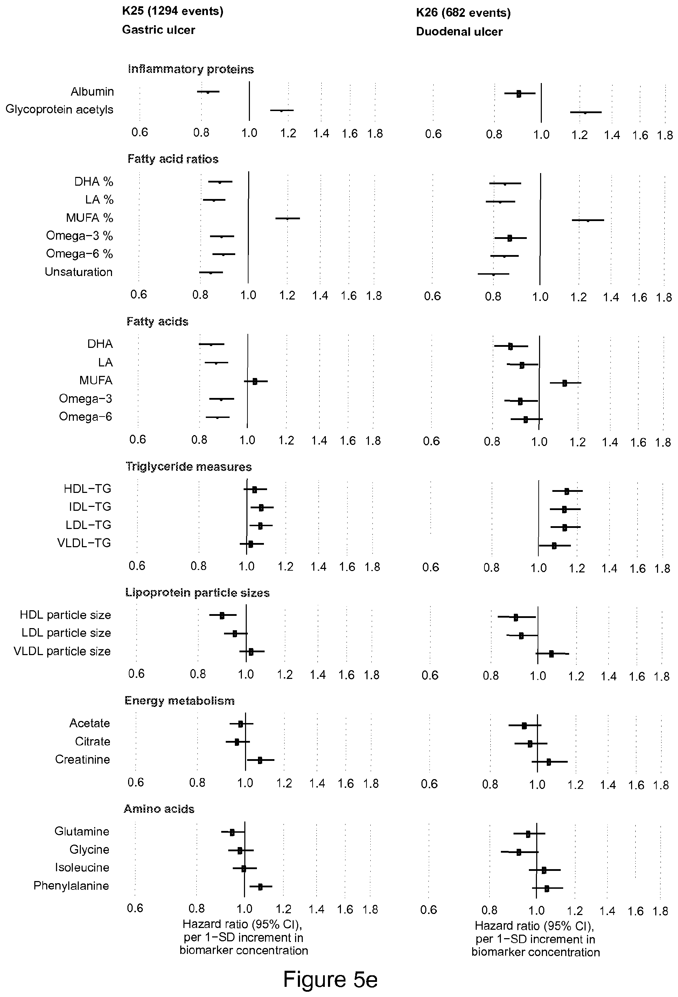

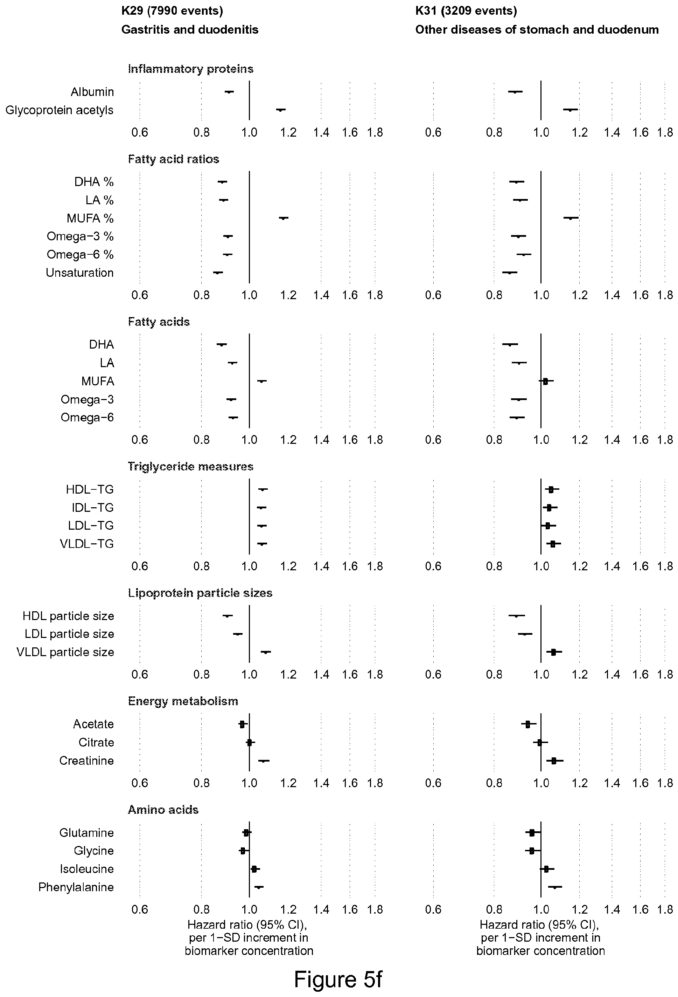

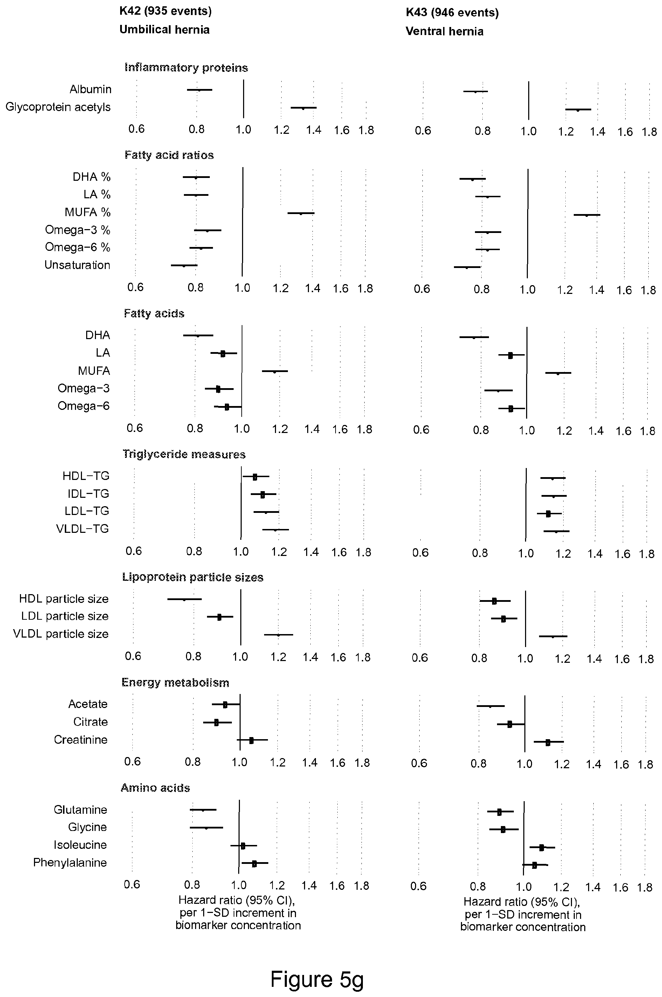

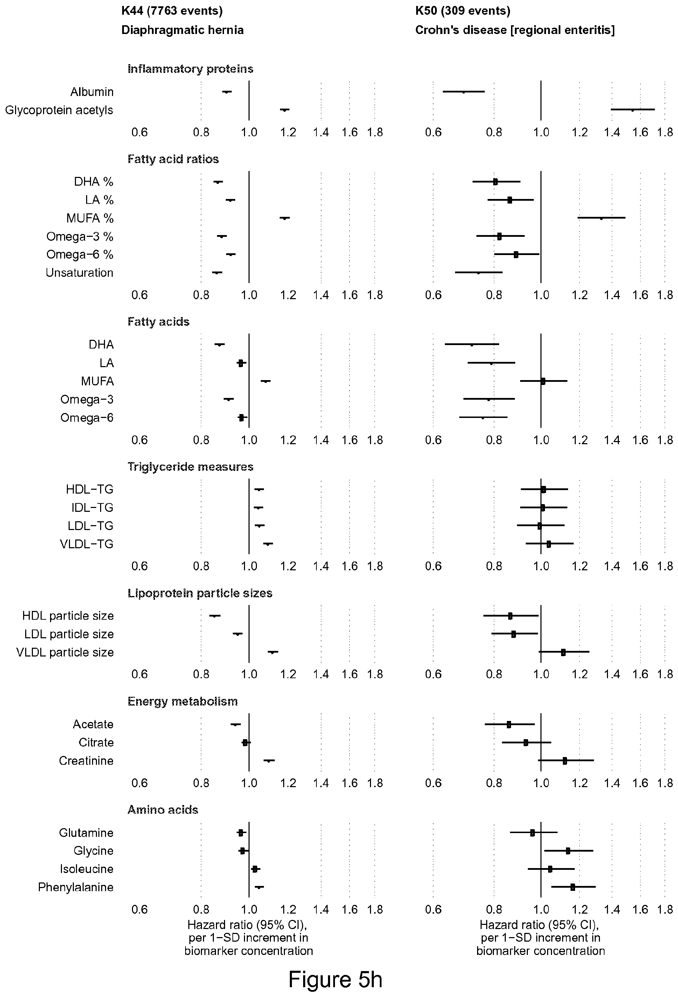

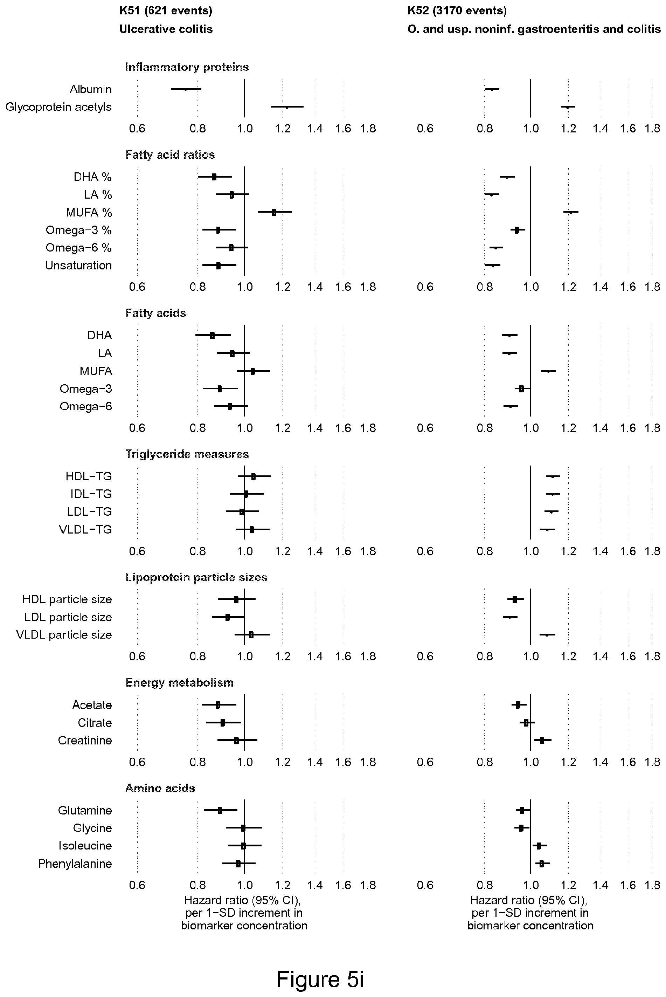

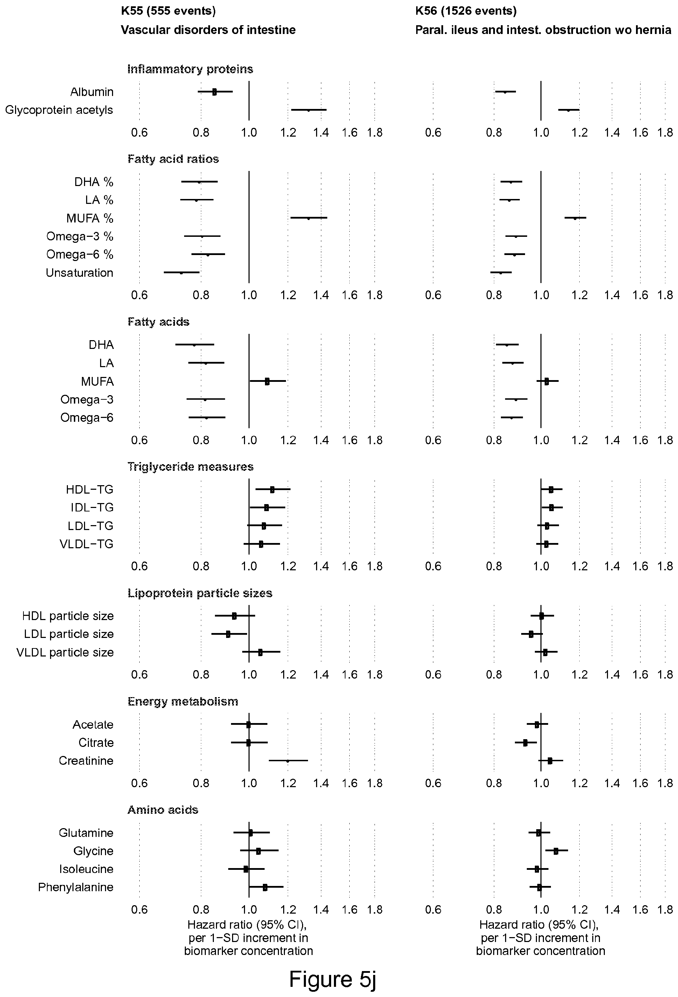

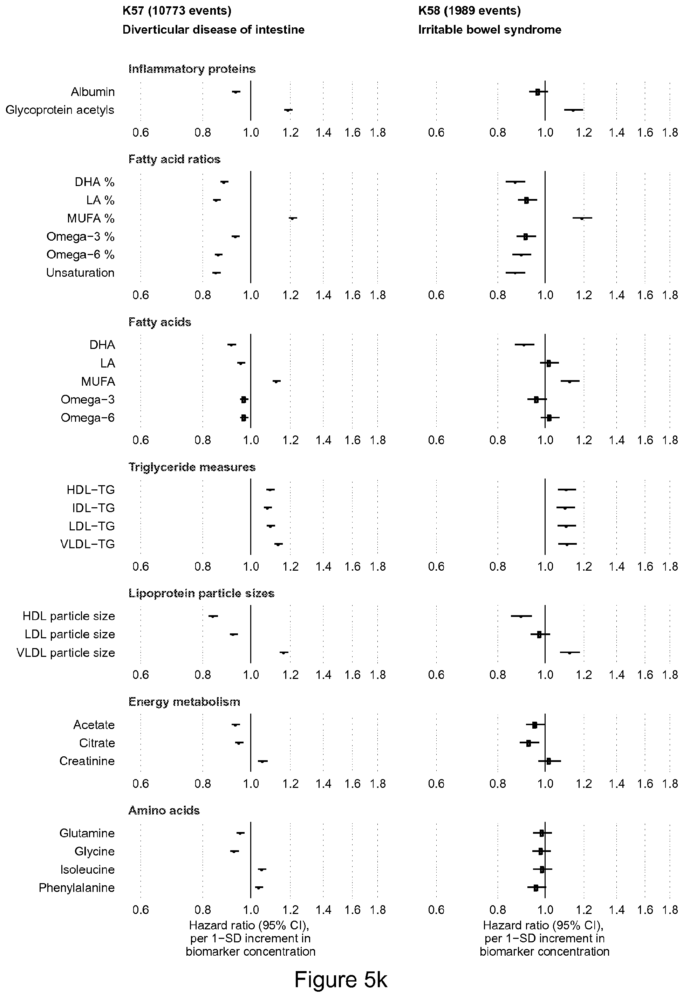

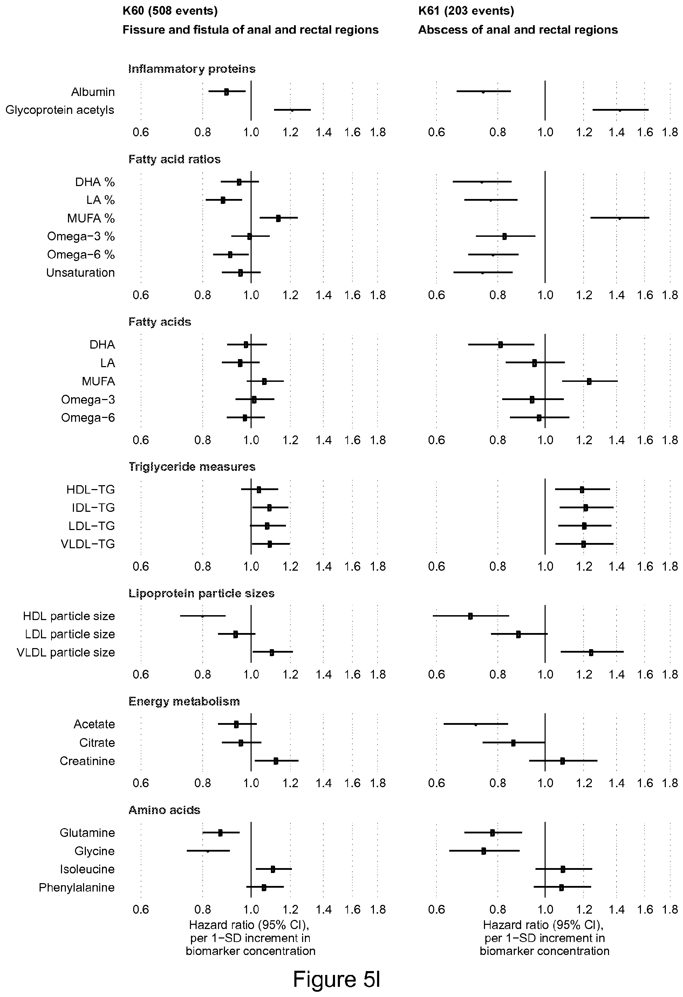

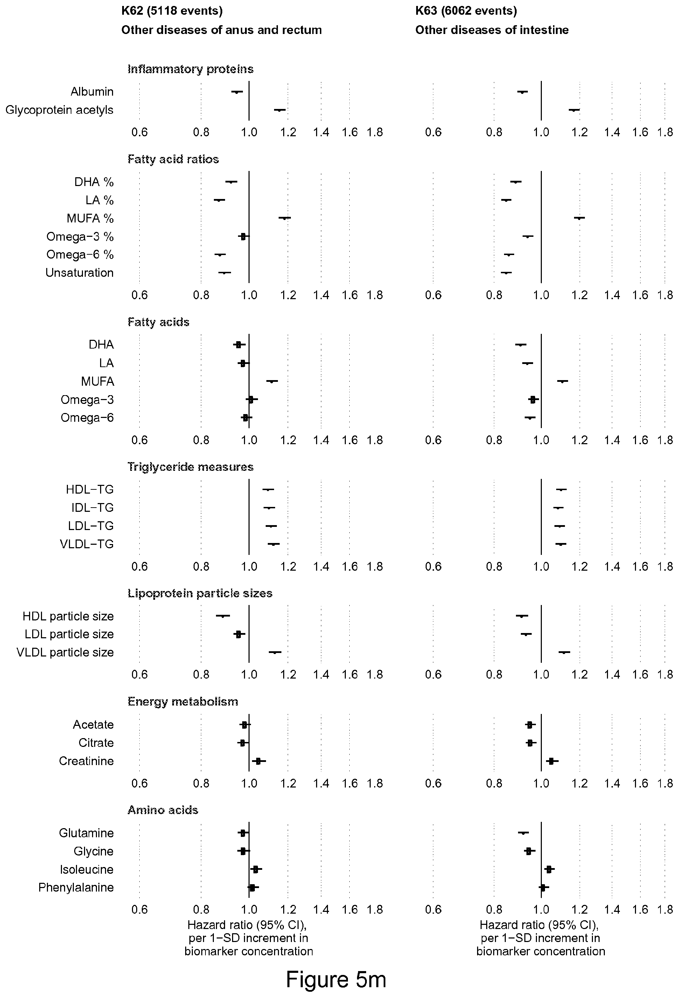

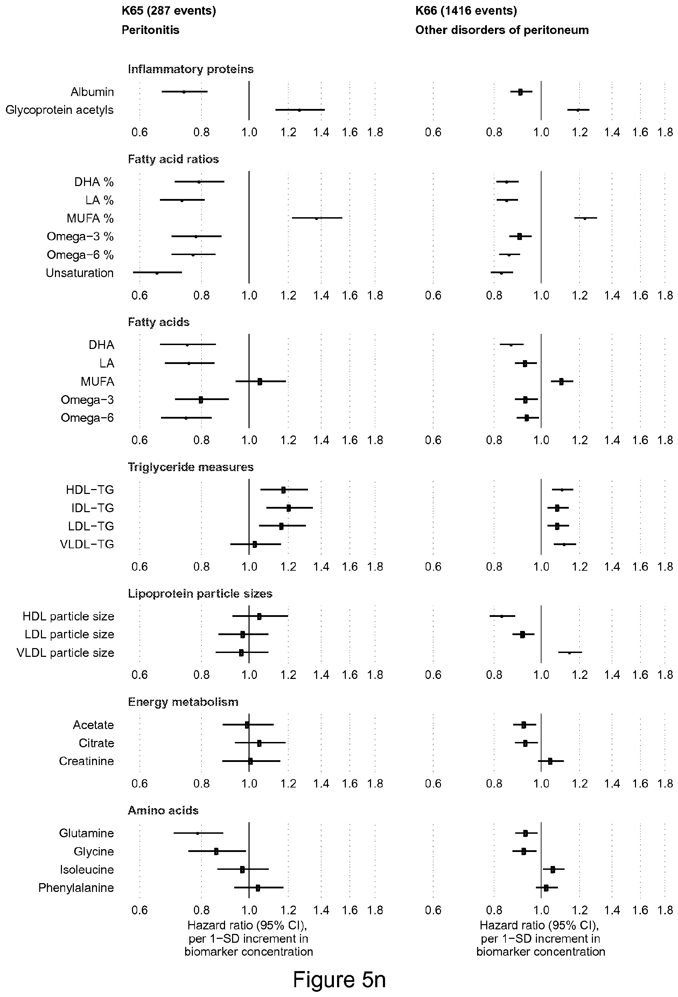

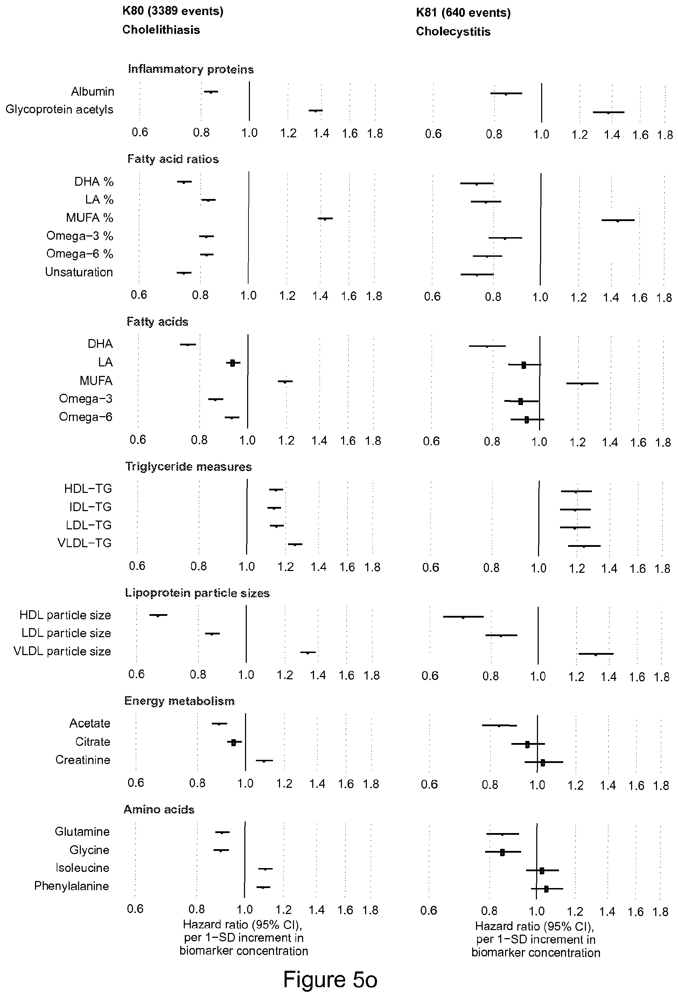

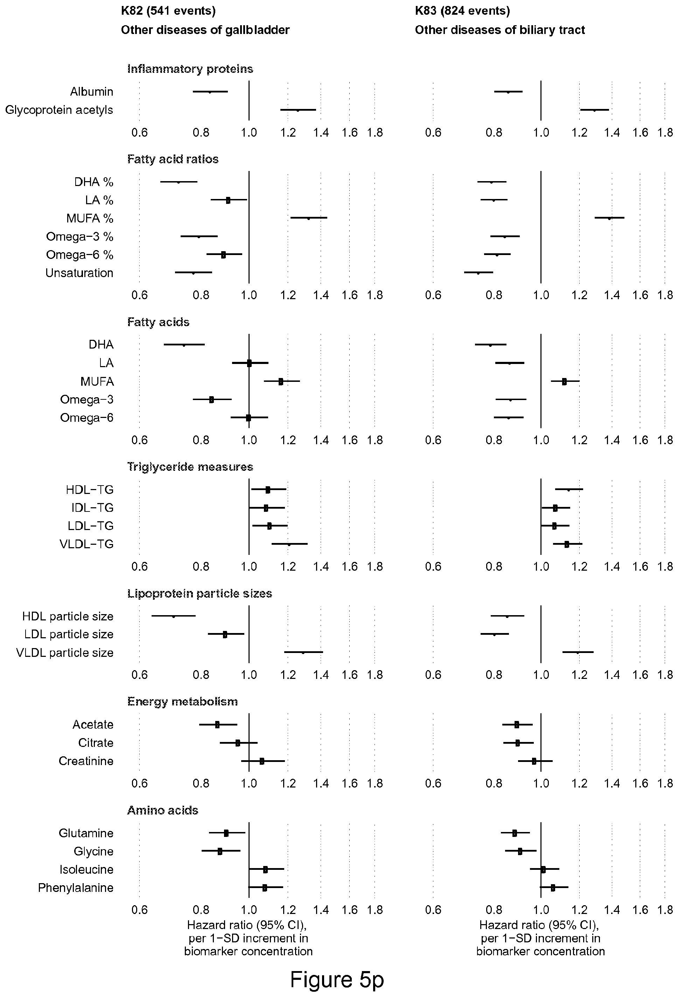

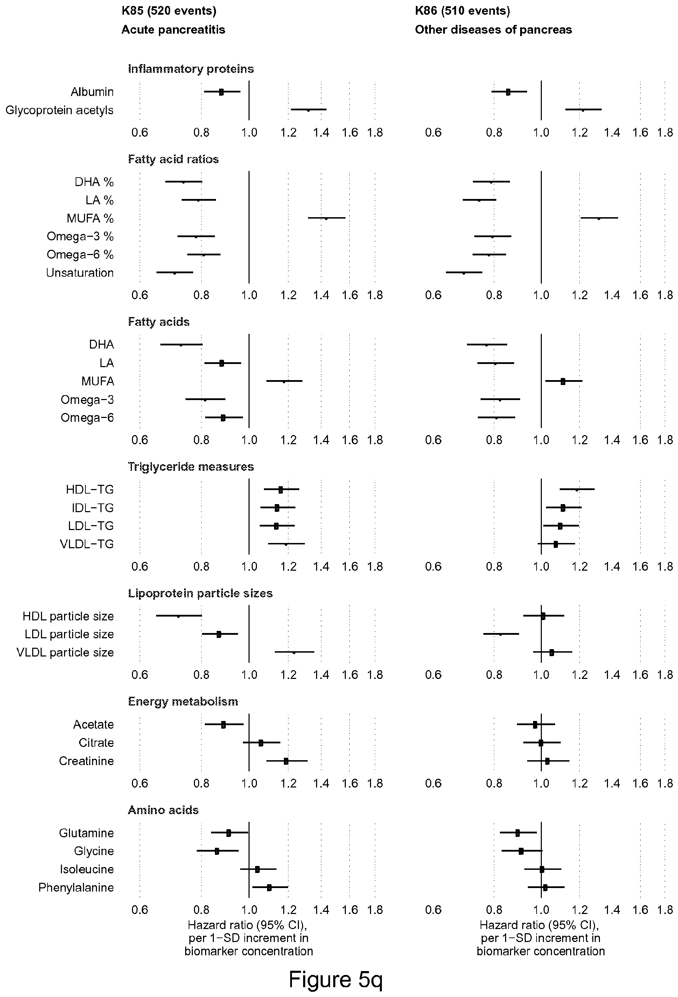

**Figures 5a-q** show the hazard ratios for the 27 blood biomarkers with future onset of each of the 34 specific digestive system diseases (defined by ICD-10 3-character diagnosis codes) studied here. The hazard ratios are shown in absolute concentrations, scaled to the standard deviation of each biomarker. The results are based on statistical analyses of over 115 000 individuals from the UK Biobank; the number of individuals who developed the specific disease during approximately 10 years of follow-up is indicated on the top of each plot. Filled circles denote that the P-value for association was P<0.0001 (corresponding to multiple testing correction), and open circles denote that the P-value for association was P≥0.0001. The analyses were adjusted for age, sex, and UK Biobank assessment centre using Cox proportional-hazard regression models.

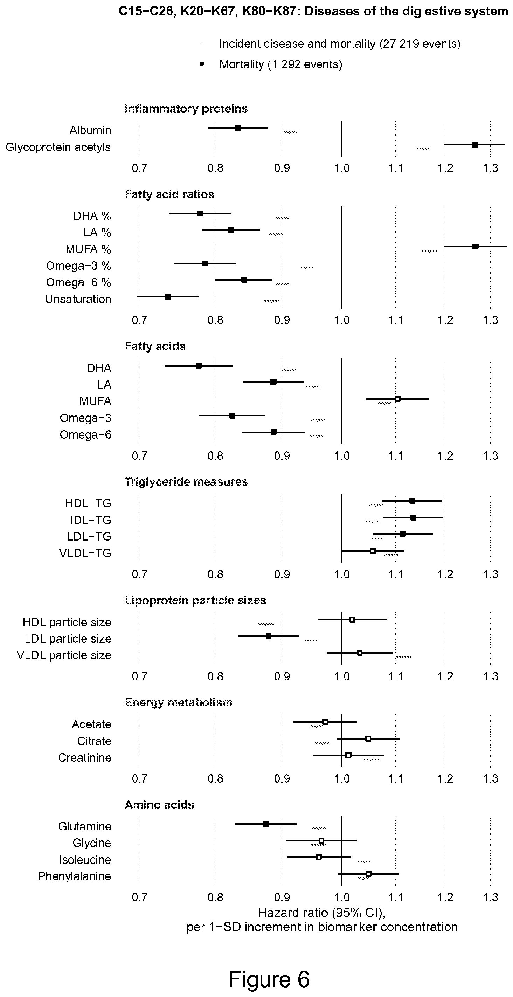

**Figure 6** shows hazard ratios for the 27 blood biomarkers with future death from 'Any Digestive System Disease' (in black). For comparison are also shown the hazard ratios for incidence of Any Digestive System Disease (i.e. combined non-fatal and fatal events, in gray). The hazard ratios are shown for biomarkers analysed in absolute concentrations, scaled to standard deviations of the study population. These results demonstrate that the biomarkers are often stronger predictors of fatal digestive system disease events. The results were similar for the biomarker associations with fatal events due to specific circulatory diseases defined by 3-character ICD-10 diagnosis codes.

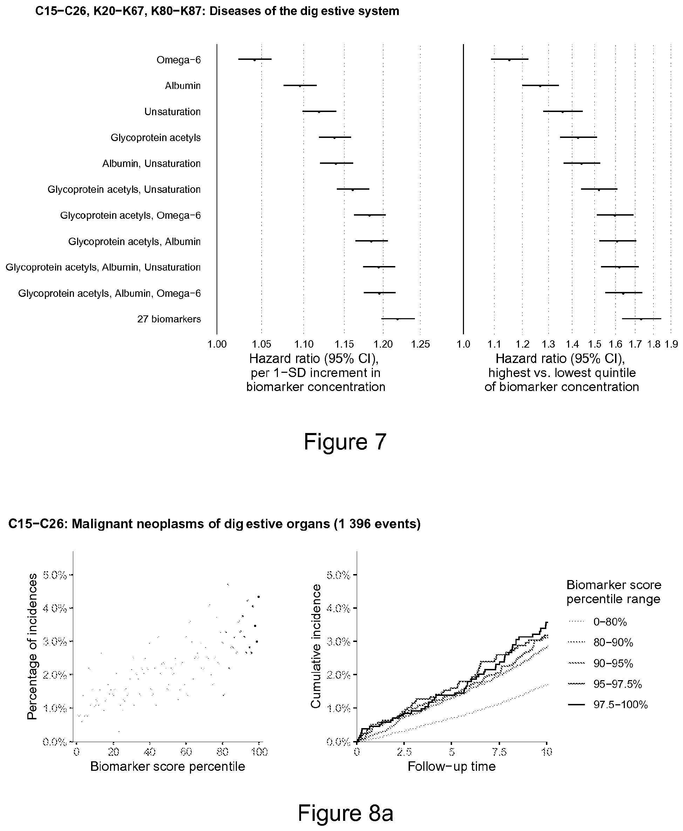

**Figure 7** shows examples of stronger association results with Any Digestive System Disease when two or more biomarkers are combined. The hazard ratios with the future risk of Any Digestive System Disease (composite endpoint of ICD-10 codes C15-C28, K20-K67 and/or K80-K87) are shown for selected combinations of pairs of biomarkers, and examples of multi-biomarker scores. The results were similar with many other combinations, in particular inclusion of different fatty acid measures in addition to albumin and glycoprotein acetyls. The multi-biomarker scores are combined in the form of $\sum_i [\beta_i * c_i] + \beta_0$ ; where $i$ is the index of summation over individual biomarkers, $\beta_i$ is the weighted coefficient attributed to biomarker $i$, $c_i$ is the blood concentration of biomarker $i$ and $\beta_0$ is an intercept term. $\beta_i$ multipliers are defined according to the multivariate association magnitude with the risk for Any Digestive System Disease, examined in the statistical analyses of the UK Biobank study for the respective combination of biomarkers. The enhancements in association magnitudes were similar for the 34 specific types of digestive system diseases listed in Table 1 as those shown here for Any Digestive System Disease.

**Illustrations of intended use: multi-biomarker scores for risk prediction of a digestive system disease**

For illustration of intended applications related to the prediction of a digestive system disease, further epidemiological analyses are illustrated below. These applications are exemplified for the prediction of the risk for malignant neoplasms of digestive organs, diseases of esophagus, stomach and/or duodenum, hernia, noninfective enteritis and/or colitis, other diseases of intestines, diseases of peritoneum and/or retroperitoneum and/or disorders of gallbladder, biliary tract and/or pancreas. Similar results apply to the other digestive system diseases listed in Table 1. Results are shown for a multi-biomarker score combining the 27 biomarkers featured in Figures 1-7. Similar results, albeit slightly weaker, are obtained with combinations of only two or three individual biomarkers.

**Figure 8a** shows the increase in the risk for malignant neoplasms of digestive organs (ICD-10 subchapter C15-C26) along with increasing levels of a multi-biomarker score composed of the weighted sum of 27 biomarkers. On the left-hand side, the risk increase is plotted in the form of gradient percentile plots, showing the proportion of individuals who developed malignant neoplasms of digestive organs during follow-up when binning individuals into the percentiles of the biomarker levels. Each dot corresponds to approximately 500 individuals. In the Kaplan-Meier plots on the right-hand side, the cumulative risk for malignant neoplasms of digestive organs during follow-up is illustrated for selected quantiles of the multi-biomarker score. Both plots serve to demonstrate that the risk is increasing in the high end of the distribution of the multi-biomarker score. The plots are shown for the validation set part of the study population, i.e. 50% which was not included for derivation of the multi-biomarker scores (n = 63 696 individuals).

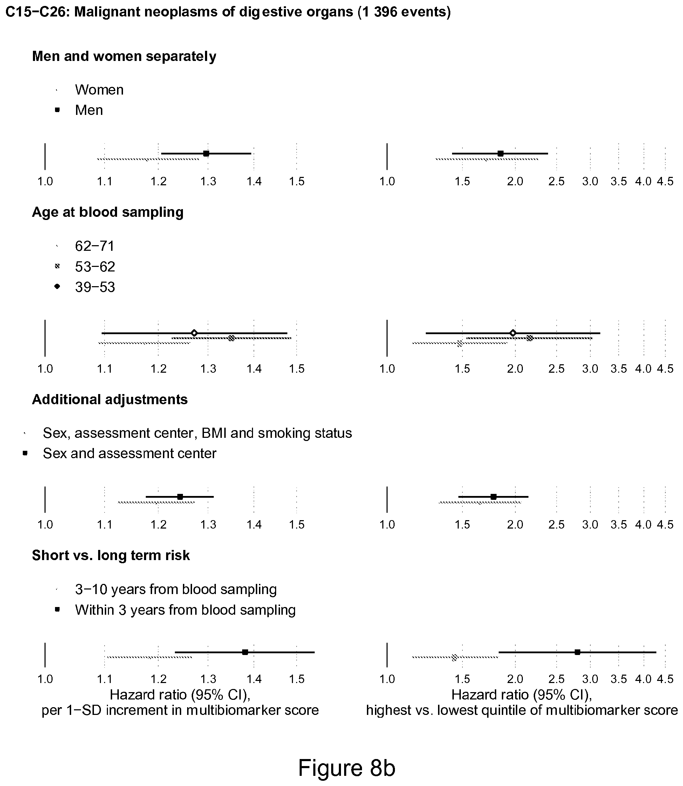

**Figure 8b** shows the hazard ratio of the same multi-biomarker score with the future onset of malignant neoplasms of digestive organs (ICD-10 subchapter C15-C26) when accounting for relevant risk factor characteristics of the study participants. The first and the second panels demonstrate that the risk prediction works effectively for both men and women as well as for people at different ages at the time of blood sampling. The third panel shows that the magnitude of the hazard ratio is only modestly attenuated when accounting for body mass index and smoking status in the statistical modelling. The last panel demontrates that the hazard ratios are substantially stronger for short-term risk prediction.

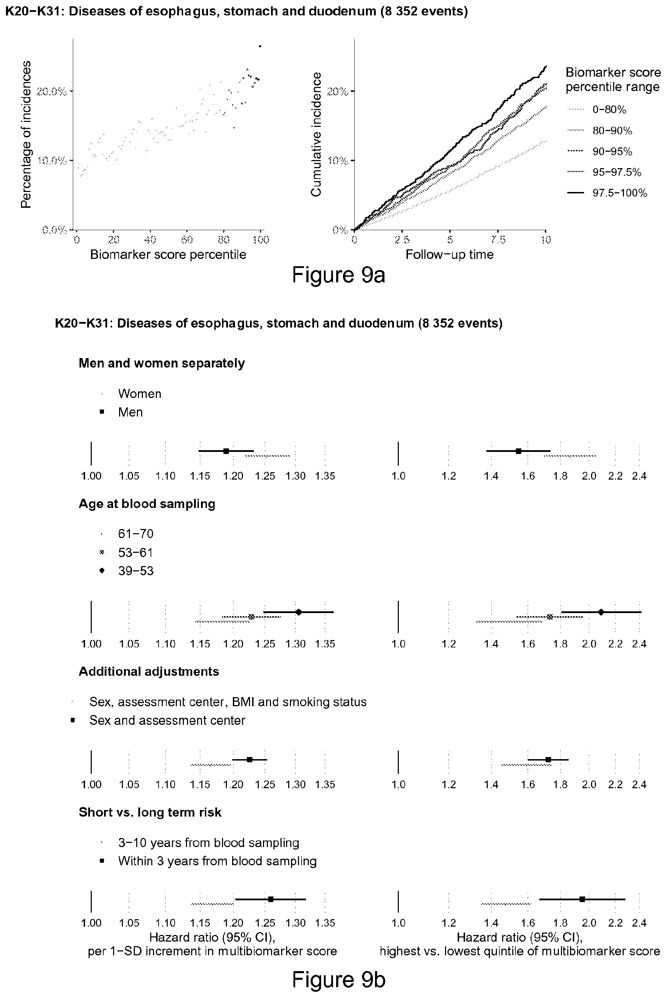

**Figure 9a** shows the increase in the risk for diseases of esophagus, stomach and duodenum (ICD-10 subchapter K20-K31) along with increasing levels of a multi-biomarker score composed of the weighted sum of 27 biomarkers. On the left-hand side, the risk increase is plotted in the form of gradient percentile plots, showing the proportion of individuals who developed diseases of esophagus, stomach and duodenum during follow-up when binning individuals into the percentiles of the biomarker levels. Each dot corresponds to approximately 500 individuals. In the Kaplan-Meier plots on the right-hand side, the cumulative risk for diseases of esophagus, stomach and duodenum during follow-up is illustrated for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included for derivation of the multi-biomarker scores (n = 55 652 individuals).

**Figure 9b** shows the hazard ratio of the same multi-biomarker score with the future onset of diseases of esophagus, stomach and duodenum (ICD-10 subchapter K20-K31) when accounting for relevant risk factor characteristics of the study participants. The first panel demonstrates that the risk prediction works effectively for both men and women. The second panel shows that the risk prediction also works for people at different ages at the time of blood sampling, with slightly stronger results for younger individuals. The third panel shows that the magnitude of the hazard ratio is only modestly attenuated when accounting for body mass index and smoking status in the statistical modelling. The last panel demonstrates that the hazard ratio is substantially stronger when focusing on short-term risk prediction.

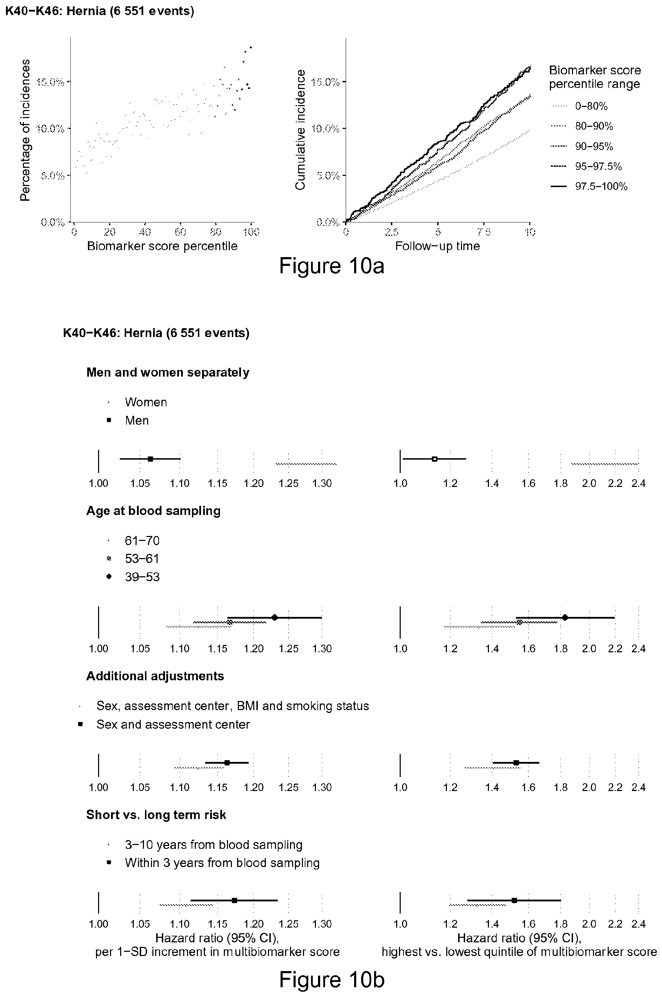

**Figure 10a** shows the increase in the risk for hernia (ICD-10 subchapter K40-K46) along with increasing levels of a multi-biomarker score composed of the weighted sum of 27 biomarkers. On the left-hand side, the risk increase is plotted in the form of gradient percentile plots, showing the proportion of individuals who developed hernia during follow-up when binning individuals into the percentiles of the biomarker levels. Each dot corresponds to approximately 500 individuals. In the Kaplan-Meier plots on the right-hand side, the cumulative risk for hernia during follow-up is illustrated for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included for derivation of the multi-biomarker scores (n = 58 330 individuals).

**Figure 10b** shows the hazard ratio of the same multi-biomarker score with the future onset of hernia (ICD-10 subchapter K40-K46) when accounting for relevant risk factor characteristics of the study participants. The first panel demonstrates that the risk prediction works effectively for both men and women, with stronger results for women. The second panel shows that the risk prediction also works for people at different ages at the time of blood sampling, with slightly stronger results for younger individuals. The third panel shows that the magnitude of the hazard ratio is only modestly attenuated when accounting for body mass index and smoking status in the statistical modelling. The last panel demonstrates that the hazard ratio is substantially stronger when focusing on short-term risk prediction.

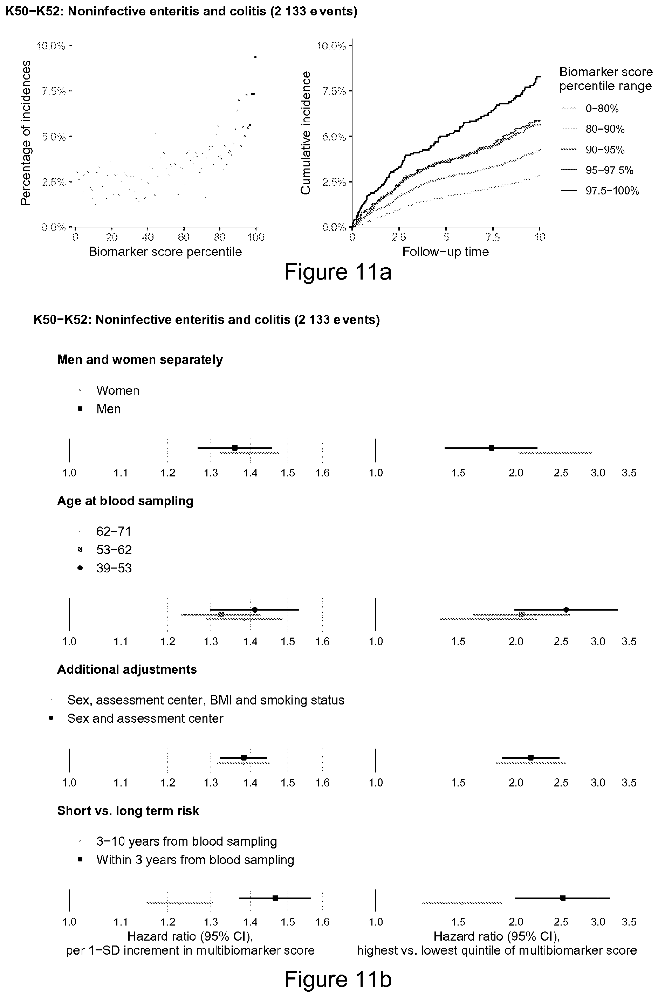

**Figure 11a** shows the increase in the risk for noninfective enteritis and colitis (ICD-10 subchapter K50-K52) along with increasing levels of a multi-biomarker score composed of the weighted sum of 27 biomarkers. On the left-hand side, the risk increase is plotted in the form of gradient percentile plots, showing the proportion of individuals who developed noninfective enteritis and colitis during follow-up when binning individuals into the percentiles of the biomarker levels. Each dot corresponds to approximately 500 individuals. In the Kaplan-Meier plots on the right-hand side, the cumulative risk for noninfective enteritis and colitis during follow-up is illustrated for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included for derivation of the multi-biomarker scores (n = 61 255 individuals).

**Figure 11b** shows the hazard ratio of the same multi-biomarker score with the future onset of noninfective enteritis and colitis (ICD-10 subchapter K50-K52) when accounting for relevant risk factor characteristics of the study participants. The first and the second panel demonstrate that the risk prediction works effectively for both men and women, as well as for people at different ages at the time of blood sampling. The third panel shows that the magnitude of the hazard ratio remains equally strong when accounting for body mass index and smoking status in the statistical modelling. The last panel demonstrates that the hazard ratio is substantially stronger when focusing on short-term risk prediction.

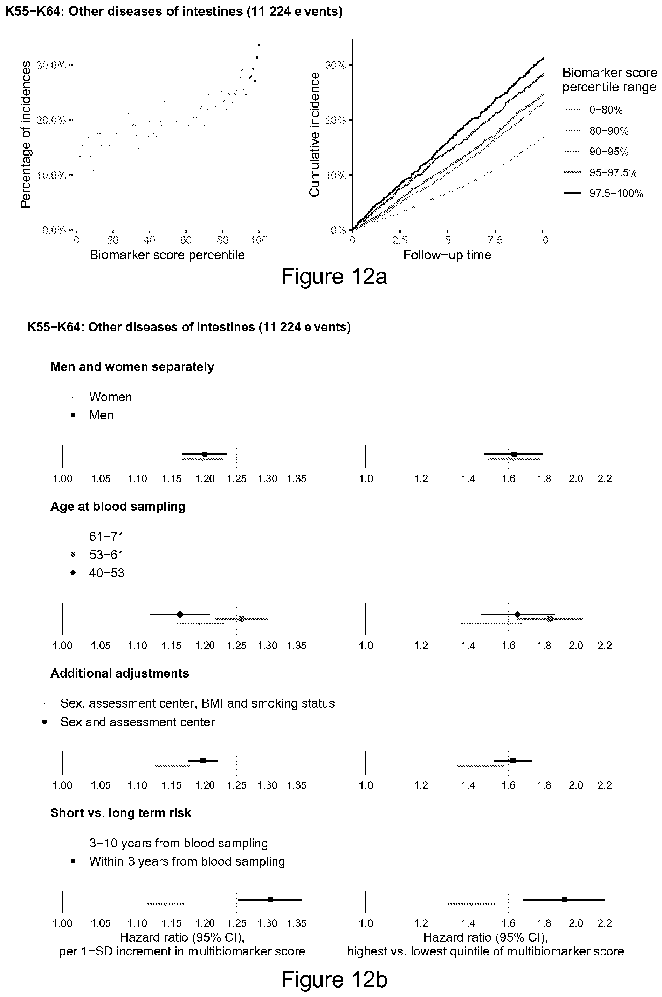

**Figure 12a** shows the increase in the risk for other diseases of intestines (ICD-10 subchapter K55-K64) along with increasing levels of a multi-biomarker score composed of the weighted sum of 27 biomarkers. On the left-hand side, the risk increase is plotted in the form of gradient percentile plots, showing the proportion of individuals who developed other diseases of intestines during follow-up when binning individuals into the percentiles of the biomarker levels. Each dot corresponds to approximately 500 individuals. In the Kaplan-Meier plots on the right-hand side, the cumulative risk for other diseases of intestines during follow-up is illustrated for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included for derivation of the multi-biomarker scores (n = 56 030 individuals).

**Figure 12b** shows the hazard ratio of the same multi-biomarker score with the future onset of other diseases of intestines (ICD-10 subchapter K55-K64) when accounting for relevant risk factor characteristics of the study participants. The first two panels demonstrate that the risk prediction works effectively for both men and women, and for people at different ages at the time of blood sampling. The third panel shows that the magnitude of the hazard ratio is only modestly attenuated when accounting for body mass index and smoking status in the statistical modelling. The last panel demontrates that the hazard ratio is substantially stronger when focusing on short-term risk prediction.

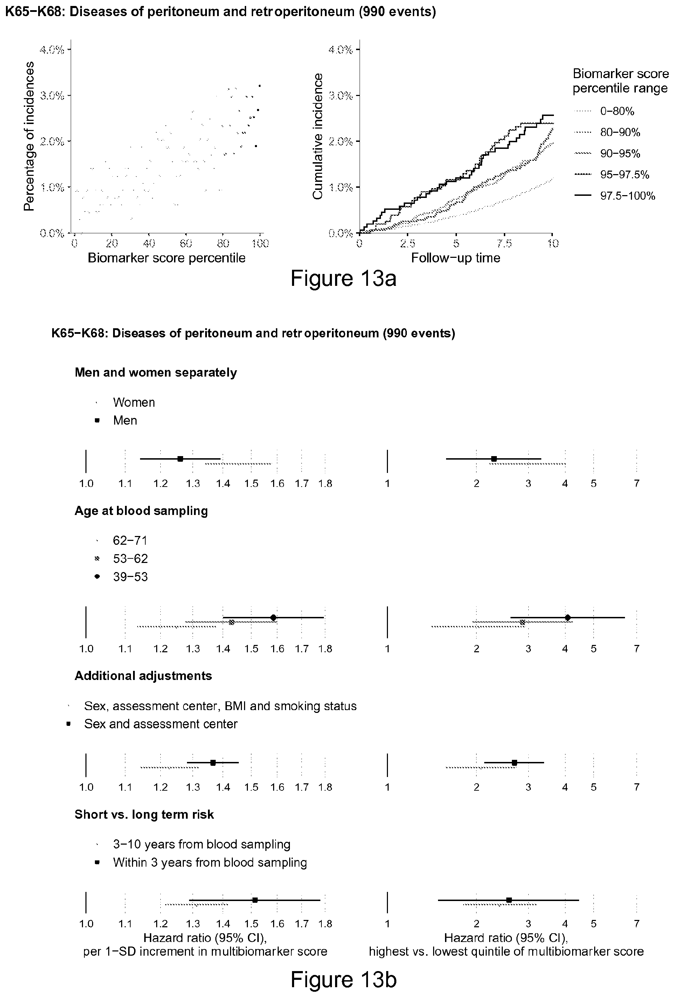

**Figure 13a** shows the increase in the risk for diseases of peritoneum and retroperteneum (ICD-10 subchapter K65-K68) along with increasing levels of a multi-biomarker score composed of the weighted sum of 27 biomarkers. On the left-hand side, the risk increase is plotted in the form of gradient percentile plots, showing the proportion of individuals who developed diseases of peritoneum and retropertoneum during follow-up when binning individuals into the percentiles of the biomarker levels. Each dot corresponds to approximately 500 individuals. In the Kaplan-Meier plots on the right-hand side, the cumulative risk for diseases of peritoneum and retropertoneum during follow-up is illustrated for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included for derivation of the multi-biomarker scores (n = 63 750 individuals).

**Figure 13b** shows the hazard ratio of the same multi-biomarker score with the future onset of diseases of peritoneum and retropertoneum (ICD-10 subchapter K65-K68) when accounting for relevant risk factor characteristics of the study participants. The first panel demonstrates that the risk prediction works effectively for both men and women. The second panel shows that risk prediction also works for people at different ages at the time of blood sampling, with stronger results for younger individuals. The third panel shows that the magnitude of the hazard ratio is only modestly attenuated when accounting for body mass index and smoking status in the statistical modelling. The last panel demonstrates that the hazard ratio is substantially stronger when focusing on short-term risk prediction.

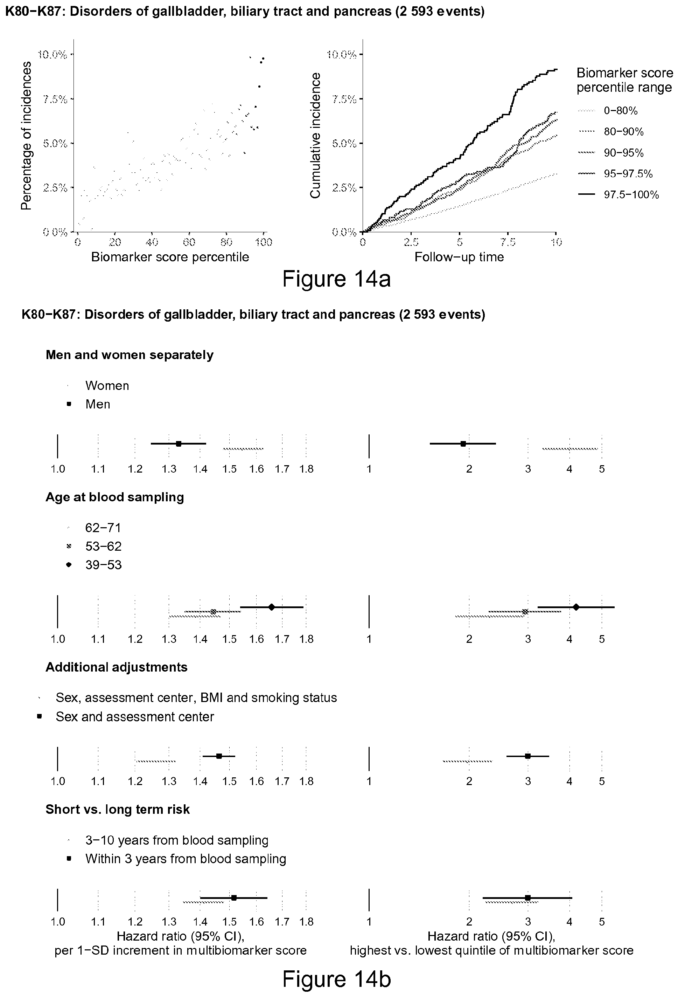

**Figure 14a** shows the increase in the risk for disorders of gallbladder, biliary tract and pancreas (ICD-10 subchapter K80-K87) along with increasing levels of a multi-biomarker score composed of the weighted sum of 27 biomarkers. On the left-hand side, the risk increase is plotted in the form of gradient percentile plots, showing the proportion of individuals who developed disorders of gallbladder, biliary tract and pancreas during follow-up when binning individuals into the percentiles of the biomarker levels. Each dot corresponds to approximately 500 individuals. In the Kaplan-Meier plots on the right-hand side, the cumulative risk for disorders of gallbladder, biliary tract and pancreas during follow-up is illustrated for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included for derivation of the multi-biomarker scores (n = 61 565 individuals).

**Figure 14b** shows the hazard ratio of the same multi-biomarker score with the future onset of disorders of gallbladder, biliary tract and pancreas (ICD-10 subchapter K80-K87) when accounting for relevant risk factor characteristics of the study participants. The first panel demonstrates that the risk prediction works effectively for both men and women, with slightly stonger results for women. The second panel shows that risk prediction also works for people at different ages at the time of blood sampling, with stronger results for younger individuals. The third panel shows that the magnitude of the hazard ratio is only modestly attenuated when accounting for body mass index and smoking status in the statistical modelling. The last panel demontrates that the hazard ratio is stronger when focusing on short-term risk prediction.

It is obvious to a person skilled in the art that with the advancement of technology, the basic idea may be implemented in various ways. The embodiments are thus not limited to the examples described above; instead they may vary within the scope of the claims.

The embodiments described hereinbefore may be used in any combination with each other. Several of the embodiments may be combined together to form a further embodiment. A method disclosed herein may comprise at least one of the embodiments described hereinbefore. It will be understood that the benefits and advantages described above may relate to one embodiment or may relate to several embodiments. The embodiments are not limited to those that solve any or all of the stated problems or those that have any or all of the stated benefits and advantages. It will further be understood that reference to 'an' item refers to one or more of those items. The term "comprising" is used in this specification to mean including the feature(s) or act(s) followed thereafter, without excluding the presence of one or more additional features or acts.

**Claims**

**1.** A method for determining whether a subject is at risk of developing a digestive system disease;

wherein the method comprises determining in a biological sample obtained from the subject a quantitative value of at least one biomarker of the following in the biological sample:

- glycoprotein acetyls,
- albumin,
- ratio of docosahexaenoic acid to total fatty acids,
- ratio of linoleic acid to total fatty acids,
- ratio of monounsaturated fatty acids and/or of oleic acid to total fatty acids,
- ratio of omega-3 fatty acids to total fatty acids,
- ratio of omega-6 fatty acids to total fatty acids,
- fatty acid degree of unsaturation,
- docosahexaenoic acid,
- linoleic acid,
- monounsaturated fatty acids and/or oleic acid,
- omega-3 fatty acids,
- omega-6 fatty acids,
- triglycerides in high-density lipoprotein (HDL),
- triglycerides in intermediate-density lipoprotein (IDL),
- triglycerides in low-density lipoprotein (LDL),
- triglycerides in very-low-density lipoprotein (VLDL),
- high-density lipoprotein (HDL) particle size,
- low-density lipoprotein (LDL) particle size,
- very-low-density lipoprotein (VLDL) particle size,
- acetate,
- citrate,
- creatinine,
- glutamine,
- glycine,
- isoleucine,
- phenylalanine; and

comparing the quantitative value(s) of the at least one biomarker to a control sample or to a control value; wherein an increase or a decrease in the quantitative value(s) of the at least one biomarker, when compared to the control sample or to the control value, is/are indicative of the subject having an increased risk of developing the digestive system disease; wherein the at least one biomarker comprises or is glycoprotein acetyls, wherein glycoprotein acetyls refers to a nuclear magnetic resonance spectroscopy signal that represents the abundance of circulating glycated proteins; and wherein the digestive system disease is cholelithiasis (ICD-10 diagnosis code K80); cholecystitis (ICD-10 diagnosis code K81); and/or other disease of gallbladder (ICD-10 diagnosis code K82).

2. The method according to claim 1, wherein the method comprises determining in the biological sample quantitative values of a plurality of biomarkers, such as two, three, four, five or more biomarkers.

3. The method according to any one of claims 1 - 2, wherein the method comprises determining in the biological sample obtained from the subject a quantitative value of the following biomarkers:

- glycoprotein acetyls;
- albumin; and
comparing the quantitative value(s) of the biomarkers to a control sample or to a control value(s); wherein an increase or a decrease in the quantitative value(s) of the biomarkers, when compared to the control sample or to the control value, is/are indicative of the subject having an increased risk of developing the digestive system disease.

4. The method according to any one of claims 1 - 3, wherein the method comprises determining in the biological sample obtained from the subject a quantitative value of the following biomarkers:

- glycoprotein acetyls,
- at least one fatty acid measure(s) of the following: ratio of docosahexaenoic acid to total fatty acids, docosahexaenoic acid, ratio of linoleic acid to total fatty acids, linoleic acid, ratio of monounsaturated fatty acids and/or of oleic acid to total fatty acids, monounsaturated fatty acids and/or oleic acid, ratio of omega-3 fatty acids to total fatty acids, omega-3 fatty acids, ratio of omega-6 fatty acids to total fatty acids, omega-6 fatty acids, fatty acid degree of unsaturation; and

comparing the quantitative value(s) of the biomarkers to a control sample or to a control value(s);

wherein an increase or a decrease in the quantitative value(s) of the biomarkers, when compared to the control sample or to the control value, is/are indicative of the subject having an increased risk of developing the digestive system disease.

5. The method according to any one of claims 1 - 4, wherein the quantitative value of the at least one biomarker is/are measured using nuclear magnetic resonance spectroscopy.

6. The method according to any one of claims 1 - 5, wherein the method further comprises determining whether the subject is at risk of developing the digestive system disease using a risk score, hazard ratio, odds ratio, and/or predicted absolute risk or relative risk calculated on the basis of the quantitative value(s) of the at least one biomarker or of the plurality of the biomarkers.

7. The method according to claim 6, wherein the risk score, hazard ratio, odds ratio, and/or predicted relative risk and/or absolute risk is calculated on the basis of at least one further measure, such as a characteristic of the subject.

8. The method according to claim 7, wherein the characteristic of the subject includes one or more of age, height, weight, body mass index, race or ethnic group, smoking, and/or family history of digestive system diseases; or age, sex, smoking status, use of alcohol, body mass index (BMI), physical activity, dietary factors such as low-fiber or high-fat diet, stress, use of certain medicines such as antacids, iron pills, strong pain medicines or laxatives, genetic risk and/or prior medical and/or family history of having digestive system diseases and/or other comorbidities.

9. The method according to any one of claims 1 - 8, wherein the method comprises determining in the biological sample obtained from the subject a quantitative value of the following biomarkers:

- glycoprotein acetyls,
- albumin,
- ratio of docosahexaenoic acid to total fatty acids,
- ratio of linoleic acid to total fatty acids,
- ratio of monounsaturated fatty acids and/or of oleic acid to total fatty acids,
- ratio of omega-3 fatty acids to total fatty acids,
- ratio of omega-6 fatty acids to total fatty acids,
- fatty acid degree of unsaturation,
- docosahexaenoic acid,
- linoleic acid,
- monounsaturated fatty acids and/or oleic acid,
- omega-3 fatty acids,
- omega-6 fatty acids,
- triglycerides in high-density lipoprotein (HDL),
- triglycerides in intermediate-density lipoprotein (IDL),
- triglycerides in low-density lipoprotein ( LDL),
- triglycerides in very-low-density lipoprotein (VLDL),
- high-density lipoprotein (HDL) particle size,
- low-density lipoprotein (LDL) particle size,
- very-low-density lipoprotein (VLDL) particle size,
- acetate,
- citrate,
- creatinine,
- glutamine,
- glycine,
- isoleucine,
- phenylalanine; and

comparing the quantitative value(s) of the biomarkers to a control sample or to a control value(s);

wherein an increase or a decrease in the quantitative value(s) of the biomarkers, when compared to the control sample or to the control value, is/are indicative of the subject having an increased risk of developing the digestive system disease.

**Patentansprüche**

1. Verfahren zur Bestimmung, ob bei einem Probanden das Risiko besteht, eine Erkrankung des Verdauungssystems zu entwickeln, wobei das Verfahren umfasst,

   Bestimmen in einer von einem Probanden entnommenen biologischen Probe eines quantitativen Werts mindestens eines der folgenden Biomarker in der biologischen Probe:

   - Glykoprotein-Acetyle,
   - Albumin,
   - Verhältnis von Docosahexaensäure zu Gesamtfettsäuren,
   - Verhältnis von Linolsäure zu Gesamtfettsäuren,
   - Verhältnis von einfach ungesättigten Fettsäuren und/oder Ölsäure zu den Gesamtfettsäuren,
   - Verhältnis von Omega-3-Fettsäuren zu den Gesamtfettsäuren,
   - Verhältnis von Omega-6-Fettsäuren zu den Gesamtfettsäuren,
   - Grad der Fettsäure-Ungesättigtheit,
   - Docosahexaensäure,
   - Linolsäure,
   - einfach ungesättigte Fettsäuren und/oder Ölsäure,
   - Omega-3-Fettsäuren,
   - Omega-6-Fettsäuren,
   - Triglyceride in Lipoproteinen hoher Dichte (HDL),
   - Triglyceride in Lipoproteinen mittlerer Dichte (IDL),
   - Triglyceride in Lipoproteinen niedriger Dichte (LDL),
   - Triglyceride in Lipoproteinen sehr niedriger Dichte (VLDL),
   - Partikelgröße von Lipoproteinen hoher Dichte (HDL),
   - Partikelgröße von Lipoproteinen niedriger Dichte (LDL),
   - Partikelgröße von Lipoproteinen sehr niedriger Dichte (VLDL),
   - Acetat,
   - Citrat,
   - Kreatinin,
   - Glutamin,
   - Glycin,
   - Isoleucin,
   - Phenylalanin; und

   Vergleichen des quantitativen Werts (der quantitativen Werte) des mindestens einen Biomarkers mit einer Kontrollprobe oder einem Kontrollwert;
   wobei eine Zunahme oder eine Abnahme des quantitativen Werts (der quantitativen Werte) des mindestens einen Biomarkers im Vergleich zur Kontrollprobe oder zum Kontrollwert darauf hinweist, dass der Proband ein erhöhtes Risiko für die Entwicklung der Erkrankung des Verdauungssystems aufweist;
   wobei der mindestens eine Biomarker Glykoprotein-Acetyle umfasst oder ist, wobei Glykoprotein-Acetyle ein Kernspinresonanzspektroskopiesignal bezeichnen, das die Häufigkeit zirkulierender glykierter Proteine darstellt; und
   wobei die Erkrankung des Verdauungssystems Cholelithiasis (ICD-10-Diagnosecode K80), Cholezystitis (ICD-10-Diagnosecode K81) und/oder eine andere Erkrankung der Gallenblase (ICD-10-Diagnosecode K82) ist.

2. Verfahren nach Anspruch 1, wobei das Verfahren das Bestimmen quantitativer Werte einer Vielzahl von Biomarkern, beispielsweise zwei, drei, vier, fünf oder mehr Biomarkern, in der biologischen Probe umfasst.

3. Verfahren nach einem der Ansprüche 1 bis 2, wobei das Verfahren umfasst

   Bestimmen eines quantitativen Werts der folgenden Biomarker in der von dem Probanden erhaltenen biologischen Probe:

   - Glykoprotein-Acetylen;
   - Albumin; und

Vergleichen des quantitativen Werts (der quantitativen Werte) der Biomarker mit einer Kontrollprobe oder einem Kontrollwert (Kontrollwerten);

wobei eine Zunahme oder Abnahme des quantitativen Werts (der quantitativen Werte) der Biomarker im Vergleich zur Kontrollprobe oder zum Kontrollwert darauf hinweist, dass der Proband ein erhöhtes Risiko für die Entwicklung der Erkrankung des Verdauungssystems aufweist.

4. Verfahren nach einem der Ansprüche 1 bis 3, wobei das Verfahren umfasst

Bestimmen eines quantitativen Werts der folgenden Biomarker in der von dem Probanden erhaltenen biologischen Probe:

- Glykoprotein-Acetyle,
- mindestens einen Fettsäurewert (Fettsäurewerte) aus den folgenden: Verhältnis von Docosahexaensäure zu Gesamtfettsäuren, Docosahexaensäure, Verhältnis von Linolsäure zu Gesamtfettsäuren, Linolsäure, Verhältnis von einfach ungesättigten Fettsäuren und/oder Ölsäure zu Gesamtfettsäuren, einfach ungesättigte Fettsäuren und/oder Ölsäure, Verhältnis von Omega-3-Fettsäuren zu Gesamtfettsäuren, Omega-3-Fettsäuren, Verhältnis von Omega-6-Fettsäuren zu Gesamtfettsäuren, Omega-6-Fettsäuren, Grad der Fettsäure-Ungesättigtheit; und

Vergleichen des quantitativen Werts (der quantitativen Werte) der Biomarker mit einer Kontrollprobe oder einem Kontrollwert (Kontrollwerten),

wobei eine Zunahme oder Abnahme des quantitativen Werts (der quantitativen Werte) der Biomarker im Vergleich zur Kontrollprobe oder zum Kontrollwert darauf hinweist, dass der Proband ein erhöhtes Risiko für die Entwicklung der Erkrankung des Verdauungssystems aufweist.

5. Verfahren nach einem der Ansprüche 1 bis 4, wobei der quantitative Wert des mindestens einen Biomarkers mittels Kernspinresonanzspektroskopie gemessen wird.

6. Verfahren nach einem der Ansprüche 1 bis 5, wobei das Verfahren ferner umfasst, Bestimmen, ob bei dem Probanden ein Risiko für die Entwicklung der Erkrankung des Verdauungssystems besteht, ein Risikowert, eine Gefährdungsquote, ein Chancenverhältnis und/oder ein vorhergesagtes absolutes Risiko oder relatives Risiko verwendet wird, das/die auf der Grundlage des quantitativen Werts (der quantitativen Werte) des mindestens einen Biomarkers oder der mehreren Biomarker berechnet wird/werden.

7. Verfahren nach Anspruch 6, wobei der Risikowert, die Gefährdungsquote, das Chancenverhältnis und/oder das vorhergesagte relative Risiko und/oder das vorhergesagte absolute Risiko auf der Grundlage mindestens einer weiteren Messgröße, beispielsweise einer Charakteristik des Probanden, berechnet werden können.

8. Das Verfahren nach Anspruch 7, wobei die Charakteristik des Probanden eines oder mehrere der folgenden Merkmale umfasst: Alter, Größe, Gewicht, Body-Mass-Index, Rasse oder ethnische Zugehörigkeit, Rauchen und/oder familiäre Vorbelastung mit Erkrankungen des Verdauungssystems; oder Alter, Geschlecht, Raucherstatus, Alkoholkonsum, Body-Mass-Index (BMI), körperliche Aktivität, Ernährungsfaktoren wie ballaststoffarme oder fettreiche Ernährung, Stress, Einnahme bestimmter Medikamente wie Antazida, Eisenpräparate, starke Schmerzmittel oder Abführmittel, genetisches Risiko und/oder frühere Erkrankungen und/oder familiäre Vorbelastung mit Erkrankungen des Verdauungssystems und/oder anderen Begleiterkrankungen.

9. Verfahren nach einem der Ansprüche 1 bis 8, wobei das Verfahren umfasst Bestimmen eines quantitativen Werts (quantitativer Werte) der folgenden Biomarker in der von dem Probanden erhaltenen biologischen Probe:

- Glykoprotein-Acetyle,
- Albumin,
- Verhältnis von Docosahexaensäure zu Gesamtfettsäuren,
- Verhältnis von Linolsäure zu Gesamtfettsäuren,
- Verhältnis von einfach ungesättigten Fettsäuren und/oder Ölsäure zu den Gesamtfettsäuren,
- Verhältnis von Omega-3-Fettsäuren zu den Gesamtfettsäuren,
- Verhältnis von Omega-6-Fettsäuren zu den Gesamtfettsäuren,
- Grad der Fettsäure-Ungesättigtheit,
- Docosahexaensäure,

- Linolsäure,
- einfach ungesättigte Fettsäuren und/oder Ölsäure,
- Omega-3-Fettsäuren,
- Omega-6-Fettsäuren,
- Triglyceride in Lipoproteinen hoher Dichte (HDL),
- Triglyceride in Lipoproteinen mittlerer Dichte (IDL),
- Triglyceride in Lipoproteinen niedriger Dichte (LDL),
- Triglyceride in Lipoproteinen sehr niedriger Dichte (VLDL),
- Partikelgröße von Lipoproteinen hoher Dichte (HDL),
- Partikelgröße von Lipoproteinen niedriger Dichte (LDL),
- Partikelgröße von Lipoproteinen sehr niedriger Dichte (VLDL),
- Acetat,
- Citrat,
- Kreatinin,
- Glutamin,
- Glycin,
- Isoleucin,
- Phenylalanin; und

Vergleichen des quantitativen Werts (der quantitativen Werte) der Biomarker mit einer Kontrollprobe oder einem Kontrollwert (Kontrollwerten);

wobei eine Zunahme oder eine Abnahme des quantitativen Werts (der quantitativen Werte) der Biomarker im Vergleich zur Kontrollprobe oder zum Kontrollwert darauf hinweist, dass der Proband ein erhöhtes Risiko für die Entwicklung der Erkrankung des Verdauungssystems aufweist.

## Revendications

1. Procédé permettant de déterminer si un sujet est à risque de développer une maladie du système digestif ;

   dans lequel le procédé comprend la détermination dans un échantillon biologique obtenu à partir du sujet d'une valeur quantitative d'au moins un biomarqueur des éléments suivants dans l'échantillon biologique :

   - les acétyles de glycoprotéines,
   - l'albumine,
   - le rapport entre l'acide docosahexaénoïque et les acides gras totaux,
   - le rapport entre l'acide linoléique et les acides gras totaux,
   - le rapport entre les acides gras mono-insaturés et/ou l'acide oléique et les acides gras totaux,
   - le rapport entre les acides gras oméga-3 et les acides gras totaux,
   - le rapport entre les acides gras oméga-6 et les acides gras totaux,
   - le degré d'insaturation en acides gras,
   - l'acide docosahexaénoïque,
   - l'acide linoléique,
   - les acides gras mono-insaturés et/ou l'acide oléique,
   - les acides gras oméga-3,
   - les acides gras oméga-6,
   - les triglycérides dans les lipoprotéines de haute densité (HDL),
   - les triglycérides dans les lipoprotéines de densité intermédiaire (IDL),
   - les triglycérides dans les lipoprotéines de basse densité (LDL),
   - les triglycérides dans les lipoprotéines de très basse densité (VLDL),
   - la taille des particules de lipoprotéines de haute densité (HDL),
   - la taille des particules de lipoprotéines de basse densité (LDL),
   - la taille des particules de lipoprotéines de très basse densité (VLDL),
   - l'acétate,
   - le citrate,
   - la créatinine,
   - la glutamine,
   - la glycine,

- l'isoleucine,
- la phénylalanine ; et

la comparaison de la ou des valeur(s) quantitative(s) de l'au moins un biomarqueur à un échantillon témoin ou à une valeur témoin ;

dans lequel une augmentation ou une diminution de la ou des valeur(s) quantitative(s) de l'au moins un biomarqueur, par rapport à l'échantillon témoin ou à la valeur témoin, est indicative du fait que le sujet présente un risque accru de développer la maladie du système digestif ;

dans lequel l'au moins un biomarqueur comprend ou est composé d'acétyles de glycoprotéines, dans lequel les acétyles de glycoprotéines font référence à un signal de spectroscopie de résonance magnétique nucléaire qui représente l'abondance de protéines glycatées en circulation ; et

dans lequel la maladie du système digestif est une cholélithiase (code de diagnostic K80 de l'ICD-10) ; une cholécystite (code de diagnostic K81 de l'ICD-10) ; et/ou une autre maladie de la vésicule biliaire (code de diagnostic K82 de l'ICD-10).

2. Procédé selon la revendication 1, dans lequel le procédé comprend la détermination dans l'échantillon biologique de valeurs quantitatives d'une pluralité de biomarqueurs, telle que deux, trois, quatre, cinq ou plus de cinq biomarqueurs.

3. Procédé selon l'une quelconque des revendications 1 à 2, dans lequel le procédé comprend la détermination dans l'échantillon biologique obtenu à partir du sujet d'une valeur quantitative des biomarqueurs suivants :

- les acétyles de glycoprotéines ;
- l'albumine ; et

la comparaison de la ou des valeur(s) quantitative(s) des biomarqueurs à un échantillon témoin ou à une ou plusieurs valeur(s) témoin(s) ;

dans lequel une augmentation ou une diminution de la ou des valeur(s) quantitative(s) des biomarqueurs, par rapport à l'échantillon témoin ou à la valeur témoin, est indicative du fait que le sujet présente un risque accru de développer la maladie du système digestif.

4. Procédé selon l'une quelconque des revendications 1 à 3, dans lequel le procédé comprend la détermination dans l'échantillon biologique obtenu à partir du sujet d'une valeur quantitative des biomarqueurs suivants :

- les acétyles de glycoprotéines,
- au moins une mesure d'acides gras des éléments suivants : le rapport entre l'acide docosahexaénoïque et les acides gras totaux, l'acide docosahexaénoïque, le rapport entre l'acide linoléique et les acides gras totaux, l'acide linoléique,

le rapport entre les acides gras mono-insaturés et/ou l'acide oléique et les acides gras totaux, les acides gras mono-insaturés et/ou l'acide oléique, le rapport entre les acides gras oméga-3 et les acides gras totaux, les acides gras oméga-3, le rapport entre les acides gras oméga-6 et les acides gras totaux, les acides gras oméga-6, le degré d'insaturation en acides gras ; et

la comparaison de la ou des valeur(s) quantitative(s) des biomarqueurs à un échantillon témoin ou à une ou plusieurs valeur(s) témoin(s) ;

dans lequel une augmentation ou une diminution de la ou des valeur(s) quantitative(s) des biomarqueurs, par rapport à l'échantillon témoin ou à la valeur témoin, est indicative du fait que le sujet présente un risque accru de développer la maladie du système digestif.

5. Procédé selon l'une quelconque des revendications 1 à 4, dans lequel la valeur quantitative de l'au moins un biomarqueur est mesurée à l'aide d'une spectroscopie de résonance magnétique nucléaire.

6. Procédé selon l'une quelconque des revendications 1 à 5, dans lequel le procédé comprend en outre la détermination du fait que le sujet est ou non à risque de développer la maladie du système digestif en utilisant un score de risque, un rapport de danger, un rapport de cotes et/ou un risque absolu ou un risque relatif prédit calculé sur la base de la ou des valeur(s) quantitative(s) de l'au moins un biomarqueur ou de la pluralité des biomarqueurs.

7. Procédé selon la revendication 6, dans lequel le score de risque, le rapport de danger, le rapport de cotes et/ou le risque absolu ou risque relatif prédit est calculé sur la base d'au moins une autre mesure, telle qu'une caractéristique du sujet.

**8.** Procédé selon la revendication 7, dans lequel la caractéristique du sujet inclut un ou plusieurs éléments parmi l'âge, la taille, le poids, l'indice de masse corporelle, la race ou le groupe ethnique, le tabagisme et/ou les antécédents familiaux de maladies du système digestif ; ou l'âge, le sexe, le tabagisme, la consommation d'alcool, l'indice de masse corporelle (IMC), l'activité physique, les facteurs alimentaires tels qu'un régime pauvre en fibres ou riche en graisses, le stress, l'utilisation de certains médicaments tels que des antiacides, des pilules de fer, des médicaments contre la douleur forte ou des laxatifs, un risque génétique et/ou des antécédents médicaux et/ou familiaux de maladies du système digestif et/ou d'autres comorbidités.

**9.** Procédé selon l'une quelconque des revendications 1 à 8, dans lequel le procédé comprend la détermination dans l'échantillon biologique obtenu à partir du sujet d'une valeur quantitative des biomarqueurs suivants :

- les acétyles de glycoprotéines,
- l'albumine,
- le rapport entre l'acide docosahexaénoïque et les acides gras totaux,
- le rapport entre l'acide linoléique et les acides gras totaux,
- le rapport entre les acides gras mono-insaturés et/ou l'acide oléique et les acides gras totaux,
- le rapport entre les acides gras oméga-3 et les acides gras totaux,
- le rapport entre les acides gras oméga-6 et les acides gras totaux,
- le degré d'insaturation en acides gras,
- l'acide docosahexaénoïque,
- l'acide linoléique,
- les acides gras mono-insaturés et/ou l'acide oléique,
- les acides gras oméga-3,
- les acides gras oméga-6,
- les triglycérides dans les lipoprotéines de haute densité (HDL),
- les triglycérides dans les lipoprotéines de densité intermédiaire (IDL),
- les triglycérides dans les lipoprotéines de basse densité (LDL),
- les triglycérides dans les lipoprotéines de très basse densité (VLDL),
- la taille des particules de lipoprotéines de haute densité (HDL),
- la taille des particules de lipoprotéines de basse densité (LDL),
- la taille des particules de lipoprotéines de très basse densité (VLDL),
- l'acétate,
- le citrate,
- la créatinine,
- la glutamine,
- la glycine,
- l'isoleucine,
- la phénylalanine ; et
la comparaison de la ou des valeur(s) quantitative(s) des biomarqueurs à un échantillon témoin ou à une ou plusieurs valeur(s) témoin(s) ;
dans lequel une augmentation ou une diminution de la ou des valeur(s) quantitative(s) des biomarqueurs, par rapport à l'échantillon témoin ou à la valeur témoin, est indicative du fait que le sujet présente un risque accru de développer la maladie du système digestif.

**C15–C26, K20–K67, K80–K87: Diseases of the digestive system**
**27 219 events**

Figure 1a

Figure 1b

Figure 2a

Figure 2b

Figure 3a

Figure 3b

**C15−C26: (2512 events)**
**Malignant neoplasms of digestive organs**

**K20−K31: (15651 events)**
**Dis. of esophagus, stomach and duodenum**

Figure 4a

**K40−K46: (11511 events)**
**Hernia**

**K50−K52: (3664 events)**
**Noninfective enteritis and colitis**

Inflammatory proteins

Albumin
Glycoprotein acetyls

0.7  0.8  0.9  1.0  1.1  1.2  1.3  1.4       0.7  0.8  0.9  1.0  1.1  1.2  1.3  1.4

Fatty acid ratios

DHA %
LA %
MUFA %
Omega−3 %
Omega−6 %
Unsaturation

0.7  0.8  0.9  1.0  1.1  1.2  1.3  1.4       0.7  0.8  0.9  1.0  1.1  1.2  1.3  1.4

Fatty acids

DHA
LA
MUFA
Omega−3
Omega−6

0.7  0.8  0.9  1.0  1.1  1.2  1.3  1.4       0.7  0.8  0.9  1.0  1.1  1.2  1.3  1.4

Triglyceride measures

HDL−TG
IDL−TG
LDL−TG
VLDL−TG

0.7  0.8  0.9  1.0  1.1  1.2  1.3  1.4       0.7  0.8  0.9  1.0  1.1  1.2  1.3  1.4

Lipoprotein particle sizes

HDL particle size
LDL particle size
VLDL particle size

0.7  0.8  0.9  1.0  1.1  1.2  1.3  1.4       0.7  0.8  0.9  1.0  1.1  1.2  1.3  1.4

Energy metabolism

Acetate
Citrate
Creatinine

0.7  0.8  0.9  1.0  1.1  1.2  1.3  1.4       0.7  0.8  0.9  1.0  1.1  1.2  1.3  1.4

Amino acids

Glutamine
Glycine
Isoleucine
Phenylalanine

0.7  0.8  0.9  1.0  1.1  1.2  1.3  1.4       0.7  0.8  0.9  1.0  1.1  1.2  1.3  1.4

Hazard ratio (95% CI),
per 1−SD increment in
biomarker concentration

Hazard ratio (95% CI),
per 1−SD increment in
biomarker concentration

# Figure 4b

**K55−K64: (20562 events)**
**Other diseases of intestines**

**K65−K68: (1643 events)**
**Diseases of peritoneum and retroperitoneum**

Hazard ratio (95% CI),
per 1−SD increment in
biomarker concentration

Figure 4c

**K80–K87: (4556 events)**
**Disorders of gallbladder, biliary tract and pancreas**

Hazard ratio (95% CI),
per 1–SD increment in
biomarker concentration

# Figure 4d

**C15 (324 events)**
**Malignant neoplasm of esophagus**

**C16 (243 events)**
**Malignant neoplasm of stomach**

Figure 5a

C18 (1065 events)                                    C19 (297 events)
Malignant neoplasm of colon                          Malignant neoplasm of rectosigmoid junction

Figure 5b

Figure 5c

**K21 (8692 events)**
**Gastro−esophageal reflux disease**

**K22 (3119 events)**
**Other diseases of esophagus**

Figure 5d

Figure 5e

**K29 (7990 events)**
**Gastritis and duodenitis**

**K31 (3209 events)**
**Other diseases of stomach and duodenum**

Figure 5f

**K42 (935 events)**
**Umbilical hernia**

**K43 (946 events)**
**Ventral hernia**

Figure 5g

EP 4 295 160 B1

Figure 5h

**K44 (7763 events)**
**Diaphragmatic hernia**

**K50 (309 events)**
**Crohn's disease [regional enteritis]**

Hazard ratio (95% CI), per 1-SD increment in biomarker concentration

**K51 (621 events)**
**Ulcerative colitis**

**K52 (3170 events)**
**O. and usp. noninf. gastroenteritis and colitis**

Figure 5i

Figure 5j

**K57 (10773 events)**
**Diverticular disease of intestine**

**K58 (1989 events)**
**Irritable bowel syndrome**

Figure 5k

**K60 (508 events)**
**Fissure and fistula of anal and rectal regions**

**K61 (203 events)**
**Abscess of anal and rectal regions**

Figure 5I

**K62 (5118 events)**

**Other diseases of anus and rectum**

**K63 (6062 events)**

**Other diseases of intestine**

Inflammatory proteins

Albumin
Glycoprotein acetyls

0.6   0.8   1.0   1.2   1.4   1.6   1.8          0.6   0.8   1.0   1.2   1.4   1.6   1.8

Fatty acid ratios

DHA %
LA %
MUFA %
Omega−3 %
Omega−6 %
Unsaturation

0.6   0.8   1.0   1.2   1.4   1.6   1.8          0.6   0.8   1.0   1.2   1.4   1.6   1.8

Fatty acids

DHA
LA
MUFA
Omega−3
Omega−6

0.6   0.8   1.0   1.2   1.4   1.6   1.8          0.6   0.8   1.0   1.2   1.4   1.6   1.8

Triglyceride measures

HDL−TG
IDL−TG
LDL−TG
VLDL−TG

0.6   0.8   1.0   1.2   1.4   1.6   1.8          0.6   0.8   1.0   1.2   1.4   1.6   1.8

Lipoprotein particle sizes

HDL particle size
LDL particle size
VLDL particle size

0.6   0.8   1.0   1.2   1.4   1.6   1.8          0.6   0.8   1.0   1.2   1.4   1.6   1.8

Energy metabolism

Acetate
Citrate
Creatinine

0.6   0.8   1.0   1.2   1.4   1.6   1.8          0.6   0.8   1.0   1.2   1.4   1.6   1.8

Amino acids

Glutamine
Glycine
Isoleucine
Phenylalanine

0.6   0.8   1.0   1.2   1.4   1.6   1.8          0.6   0.8   1.0   1.2   1.4   1.6   1.8

Hazard ratio (95% CI),
per 1−SD increment in
biomarker concentration

Hazard ratio (95% CI),
per 1−SD increment in
biomarker concentration

## Figure 5m

**K65 (287 events)**
**Peritonitis**

**K66 (1416 events)**
**Other disorders of peritoneum**

Figure 5n

Figure 5o

**K82 (541 events)**
**Other diseases of gallbladder**

**K83 (824 events)**
**Other diseases of biliary tract**

Figure 5p

Figure 5q

## C15-C26, K20-K67, K80-K87: Diseases of the dig estive system

· Incident disease and mortality (27 219 events)

■ Mortality (1 292 events)

Hazard ratio (95% CI),
per 1-SD increment in biomar ker concentration

# Figure 6

**C15−C26, K20−K67, K80−K87: Diseases of the dig estive system**

Figure 7

**C15−C26: Malignant neoplasms of dig estive organs (1 396 events)**

Figure 8a

**C15−C26: Malignant neoplasms of digestive organs (1 396 events)**

**Men and women separately**

· Women
▪ Men

**Age at blood sampling**

· 62−71
✳ 53−62
◆ 39−53

**Additional adjustments**

· Sex, assessment center, BMI and smoking status
▪ Sex and assessment center

**Short vs. long term risk**

· 3−10 years from blood sampling
▪ Within 3 years from blood sampling

Hazard ratio (95% CI),
per 1−SD increment in multibiomarker score

Hazard ratio (95% CI),
highest vs. lowest quintile of multibiomarker score

Figure 8b

**K20−K31: Diseases of esophagus, stomach and duodenum (8 352 events)**

## Figure 9a

**K20−K31: Diseases of esophagus, stomach and duodenum (8 352 events)**

## Figure 9b

**K40–K46: Hernia (6 551 events)**

Figure 10a

**K40–K46: Hernia (6 551 events)**

**Men and women separately**

· Women
▪ Men

**Age at blood sampling**

· 61–70
⊛ 53–61
♦ 39–53

**Additional adjustments**

· Sex, assessment center, BMI and smoking status
▪ Sex and assessment center

**Short vs. long term risk**

· 3–10 years from blood sampling
▪ Within 3 years from blood sampling

Hazard ratio (95% CI),
per 1–SD increment in multibiomarker score

Hazard ratio (95% CI),
highest vs. lowest quintile of multibiomarker score

Figure 10b

**K50–K52: Noninfective enteritis and colitis (2 133 events)**

Figure 11a

**K50–K52: Noninfective enteritis and colitis (2 133 events)**

**Men and women separately**

- Women
- Men

**Age at blood sampling**

- 62–71
- 53–62
- 39–53

**Additional adjustments**

- Sex, assessment center, BMI and smoking status
- Sex and assessment center

**Short vs. long term risk**

- 3–10 years from blood sampling
- Within 3 years from blood sampling

Hazard ratio (95% CI),
per 1–SD increment in multibiomarker score

Hazard ratio (95% CI),
highest vs. lowest quintile of multibiomarker score

Figure 11b

**K55–K64: Other diseases of intestines (11 224 e vents)**

Figure 12a

**K55–K64: Other diseases of intestines (11 224 e vents)**

Figure 12b

**K65−K68: Diseases of peritoneum and retroperitoneum (990 events)**

Figure 13a

**K65−K68: Diseases of peritoneum and retroperitoneum (990 events)**

**Men and women separately**

· Women
▪ Men

**Age at blood sampling**

· 62−71
❋ 53−62
◆ 39−53

**Additional adjustments**

· Sex, assessment center, BMI and smoking status
▪ Sex and assessment center

**Short vs. long term risk**

· 3−10 years from blood sampling
▪ Within 3 years from blood sampling

Hazard ratio (95% CI),
per 1−SD increment in multibiomarker score

Hazard ratio (95% CI),
highest vs. lowest quintile of multibiomarker score

Figure 13b

**K80–K87: Disorders of gallbladder, biliary tract and pancreas (2 593 events)**

Figure 14a

**K80–K87: Disorders of gallbladder, biliary tract and pancreas (2 593 events)**

Figure 14b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **TAN et al.** Proteomic-based analysis for identification of potential serum biomarkers in gallbladder cancer. *Oncology Reports*, 2011
- **WATANABE et al.** Alpha-fetoprotein-producing carcinoma of the gallbladder. *Digestive Diseases and Sciences*, 1993, vol. 38 (3), 561-564
- **KETTUNEN et al.** *Nature Genetics*, 2012, vol. 44, 269-276
- **SOININEN et al.** *Circulation: Cardiovascular Genetics*, 2015, vol. 8, 212-206
- **RITCHIE et al.** *Cell Systems*, 2015, vol. 1 (4), 293-301
- **CONNELLY et al.** *J Transl Med.*, 2017, vol. 15 (1), 219
- **KETTUNEN et al.** *Circ Genom Precis Med.*, 2018, vol. 11, e002234
- **SOININEN et al.** *Analyst*, 2009, vol. 134, 1781-1785
- **WÜRTZ et al.** *American Journal of Epidemiology*, 2017, vol. 186 (9), 1084-1096
- **JULA et al.** *Arterioscler Thromb Vasc Biol*, 2005, vol. 25, 2152-2159
- **SUDLOW et al.** *PLoS Med.*, 2015, vol. 12 (3), e1001779
- **SOININEN et al.** *Circ Cardiovasc Genet*, 2015, vol. 8, 192-206
- **WÜRTZ et al.** *Am J Epidemiol*, 2017, vol. 186, 1084-1096